(19) Europäisches Patentamt
European Patent Office
Office européen des brevets

(11) Veröffentlichungsnummer: **0 033 974**
**B1**

(12) # EUROPÄISCHE PATENTSCHRIFT

(45) Veröffentlichungstag der Patentschrift: **28.09.83**

(51) Int. Cl.³: **C 07 D 487/04**, A 61 K 31/55

(21) Anmeldenummer: **81100907.5**

(22) Anmeldetag: **09.02.81**

(54) **Benzodiazepin-Derivate, Zwischenprodukte und Verfahren für ihre Herstellung, ihre Verwendung, sowie diese enthaltende Arzneimittel.**

(30) Priorität: **08.02.80 CH 1038/80**

(43) Veröffentlichungstag der Anmeldung: **19.08.81 Patentblatt 81/33**

(45) Bekanntmachung des Hinweises auf die Patenterteilung: **28.09.83 Patentblatt 83/39**

(84) Benannte Vertragsstaaten: **BE CH DE FR GB IT LI NL SE**

(56) Entgegenhaltungen: **DE-A-2 456 311**

(73) Patentinhaber: **F. HOFFMANN-LA ROCHE & CO. Aktiengesellschaft, CH-4002 Basel (CH)**

(72) Erfinder: **Branca, Quirico, Dr., Bettingerstrasse 7, CH-4127 Birsfelden (CH)**
Erfinder: **Fischli, Albert Eduard, Prof. Dr., Am Ausserberg 20, CH-4125 Riehen (CH)**
Erfinder: **Szente, André, Dr., Baselstrasse 70, CH-4125 Riehen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 033 974 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Benzodiazepin-Derivate, Zwischenprodukte und Verfahren für ihre Herstellung, ihre Verwendung, sowie diese enthaltende Arzneimittel

Die vorliegende Erfindung betrifft Benzodiazepin-Derivate. Im speziellen betrifft sie Benzodiazepin-Derivate der allgemeinen Formel

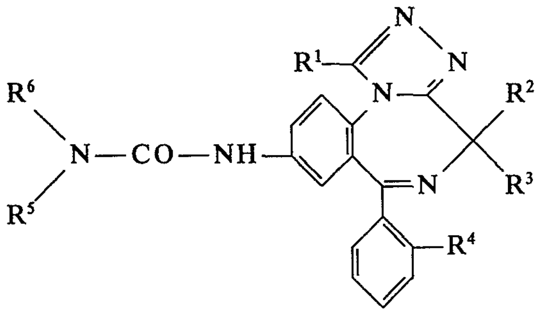

(I)

worin $R^1$ Wasserstoff oder niederes Alkyl, $R^2$ und $R^3$ je Wasserstoff oder niederes Alkyl und $R^4$ Halogen bedeuten und entweder $R^5$ Wasserstoff oder niederes Alkyl und $R^6$ niederes Alkyl oder niederes Hydroxyalkyl bedeuten oder $R^5$ und $R^6$ zusammen mit dem Stickstoffatom einen 3- bis 7gliedrigen Heterocyclus bedeuten.

Diese Verbindungen sind neu und zeichnen sich durch wertvolle pharmakodynamische Eigenschaften aus.

Gegenstand der vorliegenden Erfindung sind Benzodiazepin-Derivate der obigen allgemeinen Formel I und pharmazeutischen akzeptable Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine oder mehrere Verbindungen der allgemeinen Formel I oder pharmazeutisch akzeptable Säureadditionssalze davon und die Herstellung solcher Arzneimittel.

Der Ausdruck »niederes Alkyl«, für sich allein genommen oder in Kombinationen, wie »niederes Hydroxyalkyl« und dergleichen, bezeichnet geradkettige oder verzweigte gesättigte Kohlenwasserstoffreste mit höchstens 7, vorzugsweise höchstens 4 Kohlenwasserstoffatomen, wie Methyl, Äthyl, n-Propyl, Isopropyl, n-Butyl, tert.-Butyl usw. Der Ausdruck »niederes Hydroxyalkyl« umfaßt Reste wie 2-Hydroxyäthyl, 3-Hydroxy-2-propyl und dergleichen. Der Ausdruck »Halogen« bedeutet Fluor, Chlor, Brom oder Jod. Wenn $R^5$ und $R^6$ zusammen mit dem Stickstoffatom einen 3- bis 7gliedrigen Heterocyclus bedeuten, so kommen in erster Linie Aziridin-, Pyrrolidin- und Piperidinreste in Betracht.

Der in dieser Beschreibung verwendete Ausdruck »niederes Alkylen« bedeutet zweiwertige, gesättigte Kohlenwasserstoffreste mit höchstens 7, vorzugsweise höchstens 4 Kohlenstoffatomen, welche geradkettig oder verzweigt sein können, wie Äthylen, 1,2-Propylen und dergleichen.

Unter den Verbindungen, die von der allgemeinen Formel I umfaßt werden, sind diejenigen bevorzugt, worin $R^1$ Wasserstoff oder Methyl bedeutet. $R^2$ und $R^3$ bedeuten vorzugsweise beide Wasserstoff oder beide Methyl. $R^4$ bedeutet vorzugsweise Fluor oder Chlor. $R^5$ und $R^6$ bedeuten vorzugsweise beide Methyl oder $R^5$ Wasserstoff und $R^6$ Hydroxyäthyl oder $R^5$ und $R^6$ zusammen mit dem Stickstoffatom Pyrrolidinyl.

Ganz besonders bevorzugte, von der allgemeinen Formel I umfaßte Verbindungen im Rahmen der vorliegenden Erfindung sind:

1-[6-(o-Fluorphenyl)-1,4,4-trimethyl-4H-s-triazolo-[4,3-a] [1,4]benzodiazepin-8-yl]-3-(2-hydroxyäthyl)harnstoff;
1-[6-(o-Chlorphenyl)-1,4,4-trimethyl-4H-s-triazolo-[4,3-a] [1,4]benzodiazepin-8-yl]-3-(2-hydroxyäthyl)harnstoff;
3-[6-(o-Fluorphenyl)-4,4-dimethyl-4H-s-triazolo-[4,3-a] [1,4]benzodiazepin-8-yl]-1,1-dimethylharnstoff;
3-[6-(o-Chlorphenyl)-4H-s-triazolo[4,3-a] [1,4]-benzodiazepin-8-yl]-1,1-dimethylharnstoff;
3-[6-(o-Chlorphenyl)-1-methyl-4H-s-triazolo[4,3-a]-[1,4]benzodiazepin-8-yl]-1,1-dimethylharnstoff;
N-[6-(o-Chlorphenyl)-4,4-dimethyl-4,4-dimethyl-4H-s-triazolo-[4,3-a] [1,4]benzodiazepin-8-yl]-1-pyrrolidincarboxamid.

Weitere im Rahmen der vorliegenden Erfindung bevorzugte, von der allgemeinen Formel I umfaßte Verbindungen sind:

1-[6-(o-Fluorphenyl)-4H-s-triazolo[4,3-a] [1,4]benzodiazepin-8-yl]-3-(2-hydroxyäthyl)harnstoff;

3-[6-(o-Fluorphenyl)-4H-s-triazolo[4,3-a] [1,4]benzodiazepin-8-yl]-1,1-dimethylharnstoff;
1-[6-(o-Fluorphenyl)-1-methyl-4H-s-triazolo[4,3-a] [1,4]benzodiazepin-8-yl]-3-(2-hydroxyäthyl)harnstoff;
3-[6-(o-Fluorphenyl)-1,4,4-trimethyl-4H-s-triazolo[4,3-a] [11,4]benzodiazepin-8-yl]-1,1-dimethylharnstoff;
1-[6-(o-Chlorphenyl)-4H-s-triazolo[4,3-a] [1,4]benzodiazepin-8-yl]-3-(2-hydroxyäthyl)harnstoff;
1-[6-(o-Chlorphenyl)-1-methyl-4H-s-triazolo[4,3-a] [1,4]benzodiazepin-8-yl]-3-(2-hydroxyäthyl)harnstoff;
N-[6-(o-Chlorphenyl)-1-methyl-4H-s-triazolo[4,3-a] [1,4]benzodiazepin-8-yl]-1-pyrrolidincarboxamid;
N-[6-(o-Fluorphenyl)-1,4,4-trimethyl-4H-s-triazolo[4,3-a] [1,4]benzodiazepin-8-yl]-1-pyrrolidincarboxamid.

Die Benzodiazepin-Derivate der allgemeinen Formel I und deren pharmazeutisch akzeptable Säureadditionssalze können erfindungsgemäß dadurch hergestellt werden, daß man

a) ein Benzodiazepin-Derivat der allgemeinen Formel

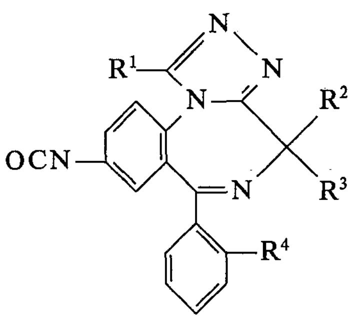

(II)

worin $R^1$, $R^2$, $R^3$ und $R^4$ obige Bedeutung besitzen, mit einer Aminoverbindung der allgemeinen Formel

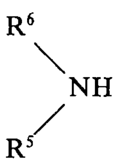

(III)

worin $R^5$ und $R^6$ obige Bedeutung besitzen, umsetzt, oder

b) ein Benzodiazepin-Derivat der allgemeinen Formel

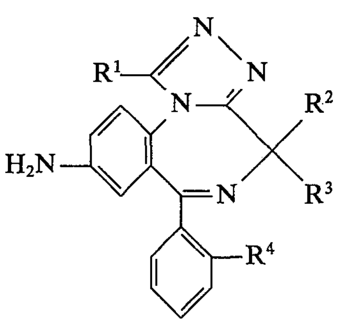

(IV)

worin $R^1$, $R^2$, $R^3$ und $R^4$ obige Bedeutung besitzen, mit einem Halogenid der allgemeinen Formel

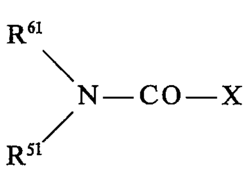

(V)

worin X Halogen bedeutet und entweder $R^{51}$ und $R^{61}$ je niederes Alkyl bedeuten oder $R^{51}$ und $R^{61}$ zusammen mit dem Stickstoffatom einen 3- bis 7gliedrigen Heterocyclus bedeuten, umsetzt oder

c) ein Benzodiazepin-Derivat der obigen allgemeinen Formel IV mit einem Isocyanat der allgemeinen Formel

$$R^{62}—NCO \qquad (VI)$$

worin $R^{62}$ niederes Alkyl bedeutet, umsetzt oder

d) aus einem Benzodiazepin-Derivat der allgemeinen Formel

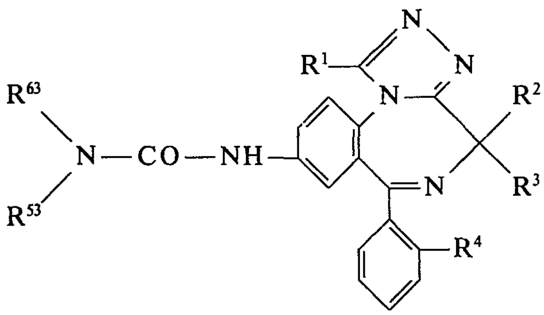

(VII)

worin $R^1$, $R^2$, $R^3$ und $R^4$ obige Bedeutung besitzen und entweder $R^{53}$ eine Schutzgruppe und $R^{63}$ niederes Alkyl oder einen Rest der Formel

$$—A—O—Y \qquad (VIII)$$

bedeuten, wobei A niederes Alkylen und Y eine Schutzgruppe bedeuten, oder $R^{53}$ Wasserstoff oder niederes Alkyl und $R^{63}$ einen Rest der obigen Formel (VIII) bedeuten, die Schutzgruppe(n) entfernt, oder

e) ein Benzodiazepin-Derivat der allgemeinen Formel

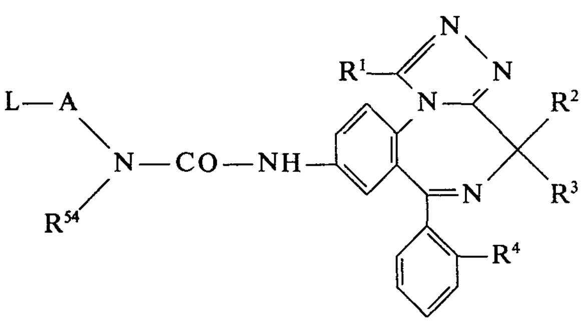

(IX)

worin $R^1$, $R^2$, $R^3$, $R^4$ und A obige Bedeutung besitzen und $R^{54}$ Wasserstoff oder niederes Alkyl und L eine Abgangsgruppe bedeuten,

f) in einem Benzodiazepin-Derivat der allgemeinen Formel

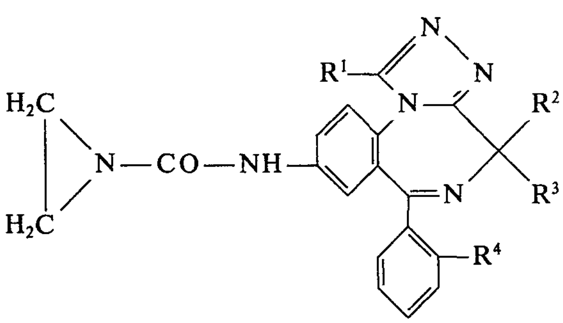

(Ia)

worin $R^1$, $R^2$, $R^3$ und $R^4$ obige Bedeutung besitzen, den Aziridinring hydrolytisch öffnet, oder

g) ein Benzodiazepin-Derivat der allgemeinen Formel I in ein pharmazeutisch akzeptables Säureadditionssalz überführt.

Nach einem ersten Verfahrensaspekt können die Benzodiazepin-Derivate der allgemeinen Formel I erfindungsgemäß aus Benzodiazepin-Derivaten der allgemeinen Formel II und Aminoverbindungen der allgemeinen Formel III hergestellt werden. Zweckmäßigerweise geht man dabei so vor, daß man das zum Einsatz vorgesehene Benzodiazepin-Derivat der allgemeinen Formel II kurz oder unmittelbar vor der Umsetzung mit der Aminoverbindung der allgemeinen Formel III auf die weiter unten beschriebene Weise aus einem entsprechenden Benzodiazepin-Derivat der allgemeinen Formel IV herstellt und das betreffende Benzodiazepin-Derivat der allgemeinen Formel II nicht in isolierter Form in die Reaktion einbringt, sondern in der Lösung, in welcher es vorgängig aus dem entsprechenden Benzodiazepin-Derivat der allgemeinen Formel IV hergestellt worden ist.

Zu der erwähnten, das Benzodiazepin-Derivat der allgemeinen Formel II enthaltenden Lösung kann man dann eine Aminoverbindung der allgemeinen Formel III geben. Hierbei kann die Aminoverbindung der allgemeinen Formel III in Form einer Lösung oder auch ohne Lösungsmittel verwendet werden; falls es sich um eine bei Raumtemperatur gasförmige Aminoverbindung handelt (was beispielsweise für Methylamin zutrifft) kann man sie als Gas in die erwähnte, das Benzodiazepin-Derivat der allgemeinen Formel II enthaltende Lösung einleiten.

Andererseits ist es auch möglich, die Aminoverbindung der allgemeinen Formel III vorzulegen zweckmäßigerweise in Form einer Lösung, und dann die erwähnte, das Benzodiazepin-Derivat der allgemeinen Formel II enthaltende Lösung zuzugeben. In vielen Fällen ist es zweckmäßig, die Aminoverbindung der allgemeinen Formel III im Überschuß einzusetzen.

Als Lösungsmittel für den vorliegenden Verfahrensaspekt eignen sich verschiedene, unter den Reaktionsbedingungen inerte organische Lösungsmittel, beispielsweise halogenierte Kohlenwasserstoffe, wie Dichloräthan, Methylenchlorid, Chloroform, o-Dichlorbenzol; Äther, wie Tetrahydrofuran, Dioxan, Dimethoxyäthan, Diäthylenglykoldimethyläther.

Die Umsetzung der Verbindungen der Formeln II und III erfolgt zweckmäßigerweise bei Raumtemperatur oder Temperaturen unterhalb davon. Es ist noch zu beachten, daß man dann, wenn man eine Lösung des Benzodiazepin-Derivats der allgemeinen Formel II vorlegt, die Aminoverbindung der allgemeinen Formel III innerhalb kurzer Zeit zugeben sollte, wogegen im umgekehrten Fall, d. h. wenn man die Aminoverbindung der allgemeinen Formel III vorlegt und dann die Lösung des Benzodiazepin-Derivats der allgemeinen Formel II zugibt, die Geschwindigkeit der Zugabe keine wesentliche Rolle spielt.

Nach einem zweiten Verfahrensaspekt können Benzodiazepin-Derivate der allgemeinen Formel I erfindungsgemäß dadurch hergestellt werden, daß man ein Benzodiazepin-Derivat der allgemeinen Formel IV mit einem Halogenid der allgemeinen Formel V umsetzt. Die Umsetzung der Verbindungen der Formeln IV und V erfolgt in Gegenwart eines säurebindenden Mittels, beispielsweise einer anorganischen Base, wie Kaliumcarbonat, Natriumcarbonat, oder einer organischen Base, z. B. einer tertiären Aminoverbindung, wie Triäthylamin, N-Äthyl-diisopropylamin, Chinuclidin.

Die Umsetzung der Verbindungen der Formeln IV und V erfolgt zweckmäßigerweise bei Raumtemperatur oder unterhalb davon; sie verläuft ziemlich langsam und dauert in der Regel mehrere Tage.

Nach einem weiteren Verfahrensaspekt können Benzodiazepin-Derivate der allgemeinen Formel I erfindungsgemäß dadurch hergestellt werden, daß man ein Benzodiazepin-Derivat der allgemeinen Formel IV mit einem Isocyanat der allgemeinen Formel VI umsetzt. Diese Umsetzung erfolgt zweckmäßigerweise in einem unter den Reaktionsbedingungen inerten organischen Lösungsmittel, beispielsweise in einem halogenierten Kohlenwasserstoff, wie Methylenchlorid, Dichloräthan, Chloroform, o-Dichlorbenzol; in einem Äther, wie Tetrahydrofuran, Dioxan, Dimethoxyäthan, Diäthylenglykoldimethyläther. In manchen Fällen erweist es sich als günstig, die Reaktion in Gegenwart einer katalytisch wirkenden geringen Menge einer Base durchzuführen, beispielsweise in Gegenwart einer tertiären Aminoverbindung, wie Triäthylamin, N-Äthyldiisopropylamin, Chinuclidin. Die Temperatur ist für die Umsetzung der Verbindungen der Formeln IV und VI nicht kritisch, und man kann demnach bei Raumtemperatur, unterhalb der Raumtemperatur oder oberhalb der Raumtemperatur arbeiten, beispielsweise bei Rückflußtemperatur.

Nach einem weiteren Verfahrensaspekt können Benzodiazepin-Derivate der allgemeinen Formel I erfindungsgemäß dadurch hergestellt werden, daß man aus einem Benzodiazepin-Derivat der allgemeinen Formel VII die Schutzgruppe bzw. die Schutzgruppen entfernt. Als Stickstoff-Schutzgruppen eignen sich für die Zwecke der vorliegenden Erfindung in erster Linie Acylgruppen, vorzugsweise leicht abspaltbare Alkoxycarbonyl- oder Aralkoxycarbonylgruppen, insbesondere die tert.-Butoxycarbonylgruppe, die Benzyloxycarbonylgruppe, ferner auch leicht abspaltbare Aralkylgruppen, wie die Benzylgruppe. Als Sauerstoff-Schutzgruppen eignen sich einerseits Acylgruppen oder Aralkylgruppen, wie sie soeben als Stickstoff-Schutzgruppen erwähnt worden sind, andererseits aber auch Ketal-Schutzgruppen, wie Tetrahydropyranyl, 2-Methoxy-2-propyl- Methoxymethyl, β-Methoxyäthoxy-methyl, leicht abspaltbare Alkylgruppen, wie tert.-Butyl, oder Alkanoylgruppen, wie Acetyl.

Die Entfernung der Schutzgruppe bzw. der Schutzgruppen aus den Benzodiazepin-Derivaten der allgemeinen Formel VII erfolgt nach an sich bekannten Methoden, wobei natürlich die Natur der zu entfernenden Schutzgruppe bzw. Schutzgruppe für die Wahl der zur Anwendung gelangenden

Methode bzw. Methoden in Betracht gezogen werden muß. Ebenfalls zu beachten ist natürlich, daß nur solche Methoden verwendet werden können, welche die Schutzgruppe bzw. Schutzgruppen selektiv entfernen, ohne daß andere im Molekül vorhandene Strukturelemente in Mitleidenschaft gezogen werden.

Die weiter oben als Beispiele für in Betracht kommende Schutzgruppen erwähnten Reste können je nach ihrer Natur hydrogenolytisch und/oder hydrolytisch abgespalten werden. So können z. B. die Benzyloxycarbonylgruppe und die tert.-Butoxycarbonylgruppe unter selektiven sauren Bedingungen abgespalten werden, z. B. durch Behandeln mit einem Gemisch von Bromwasserstoff und Eisessig, ebenso durch Behandeln mit Bortrifluorid oder Bortribromid in einem inerten organischen Lösungsmittel, wie Dichlormethan. Die tert.-Butoxycarbonylgruppe kann auch durch Behandeln mit Chlorwasserstoff in einem inerten organischen Lösungsmittel, wie Dioxan, Tetrahydrofuran, oder durch Behandeln mit Trifluoressigsäure abgespalten werden. Die Tetrahydropyranylgruppe läßt sich unter milden sauren Bedingungen abspalten, beispielsweise durch Behandeln mit verdünnter wäßriger Mineralsäure unter milden Bedingungen. Die tert.-Butylgruppe kann z. B. mittels Trifluoressigsäure abgespalten werden. Die Benzylgruppe kann durch katalytische Hydrierung, z. B. über Palladium/Kohle entfernt werden. Die Acetylgruppe kann unter milden alkalischen Bedingungen abgespalten werden, z. B. mit einer Lösung eines Natriumalkoholats im entsprechenden Alkohol (wie methanolisches Natriummethylat).

Nach einem weiteren Verfahrensaspekt können Benzodiazepin-Derivate der allgemeinen Formel I erfindungsgemäß dadurch hergestellt werden, daß man Benzodiazepin-Derivate der allgemeinen Formel IX in entsprechende Hydroxyverbindungen überführt. Die in Formel IX durch das Symbol L wiedergegebene Abgangsgruppe kann ein Halogenatom sein, insbesondere Chlor, Brom oder Jod; es können jedoch ohne weiteres auch äquivalente andere Abgangsgruppen verwendet werden, z. B. Arylsulfonyloxyreste, wie Tosyloxy, Alkylsulfonyloxyreste, wie Mesyloxy, quartäre Ammoniumgruppen, wie der Trimethylammoniumrest.

Die Überführung eines Benzodiazepin-Derivats der allgemeinen Formel IX in die entsprechende Hydroxyverbindung kann z. B. durch Solvolyse in einem wasserhaltigen System erfolgen, zweckmäßigerweise in einem Gemisch eines aromatischen Kohlenwasserstoffes, wie Benzol, und Wasser, in Gegenwart eines quartären Ammoniumsalzes, wie Tetrabutylammoniumbromid, und bei einer Temperatur zwischen Raumtemperatur und Rückflußtemperatur des Reaktionsgemisches.

Nach einem weiteren Verfahrensaspekt können Benzodiazepin-Derivate der allgemeinen Formel I dadurch erhalten werden, daß man in Benzodiazepin-Derivaten der allgemeinen Formel Ia den Aziridinring hydrolytisch öffnet. Diese hydrolytische Ringöffnung erfolgt unter sauren Bedingungen, wobei nur Säuren in Betracht kommen, deren Anion mit dem Aziridinring nicht reagiert. Man arbeitet zweckmäßigerweise in Gegenwart eines geeigneten, unter den Reaktionsbedingungen inerten organischen Lösungsmittels und bei Raumtemperatur. Beispielsweise kann man so vorgehen, daß man die Verbindung der Formel Ia in Dioxan löst, wenig Mineralsäure (z. B. einige Tropfen 25proz. Schwefelsäure) zugibt und das Gemisch während nicht allzu langer Zeit (z. B. 15 bis 30 Minuten) stehen läßt.

Nach einem weiteren Verfahrensaspekt können die Benzodiazepin-Derivate der allgemeinen Formel I erfindungsgemäß in pharmazeutisch akzeptable Säureadditionssalze übergeführt werden. Die Herstellung von solchen pharmazeutisch akzeptablen Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Citrate, Acetate, Succinate, Methansulfonate, p-Toluolsulfonate.

Die als Ausgangsstoffe verwendeten Benzodiazepin-Derivate der allgemeinen Formel II können — wie bereits weiter oben erwähnt — aus entsprechenden Benzodiazepin-Derivaten der allgemeinen Formel IV hergestellt werden, und zwar durch Umsetzen mit Phosgen. Dabei geht man zweckmäßigerweise so vor, daß man eine Lösung von Phosgen in einem unter den Reaktionsbedingungen inerten organischen Lösungsmittel vorlegt und dann unter Kühlen eine Lösung eines Benzodiazepin-Derivats der allgemeinen Formel IV zugibt, hierauf einige Zeit unter Rückfluß zum Sieden erhitzt, dann wieder abgekühlt und schließlich die erhaltene Lösung mit einer tertiären organischen Aminoverbindung, wie Triäthylamin, basisch oder mindestens neutral stellt. Die erhaltene Lösung, enthaltend ein Benzodiazepin-Derivat der allgemeinen Formel II, kann unter Feuchtigkeitsausschluß und in der Kälte während mehreren Stunden aufbewahrt werden; sie wird — wie weiter oben ausgeführt — ohne Isolierung des darin enthaltenen Benzodiazepin-Derivats der allgemeinen Formel II direkt weiterverarbeitet.

Die Verbindungen der allgemeinen Formel II sind ebenfalls Gegenstand der vorliegenden Erfindung; als ein repräsentativer Vertreter dieser Verbindungsklasse kann das [6-(o-Fluorphenyl)-4H-s-triazolo[4,3-a] [1,4]benzodiazepin-8-yl]isocyanat genannt werden.

Die Verbindungen der allgemeinen Formel IV gehören einer an sich bekannten Verbindungsklasse an. Noch nicht spezifisch vorbeschriebene Vertreter dieser Verbindungsklasse können nach an sich bekannten und jedem Fachmann geläufigen Methoden hergestellt werden, beispielsweise ausgehend von Verbindungen der Formel X gemäß nachfolgendem Reaktionsschema, worin $R^1$, $R^2$, $R^3$ und $R^4$ die eingangs erwähnte Bedeutung besitzen:

Reaktionsschema

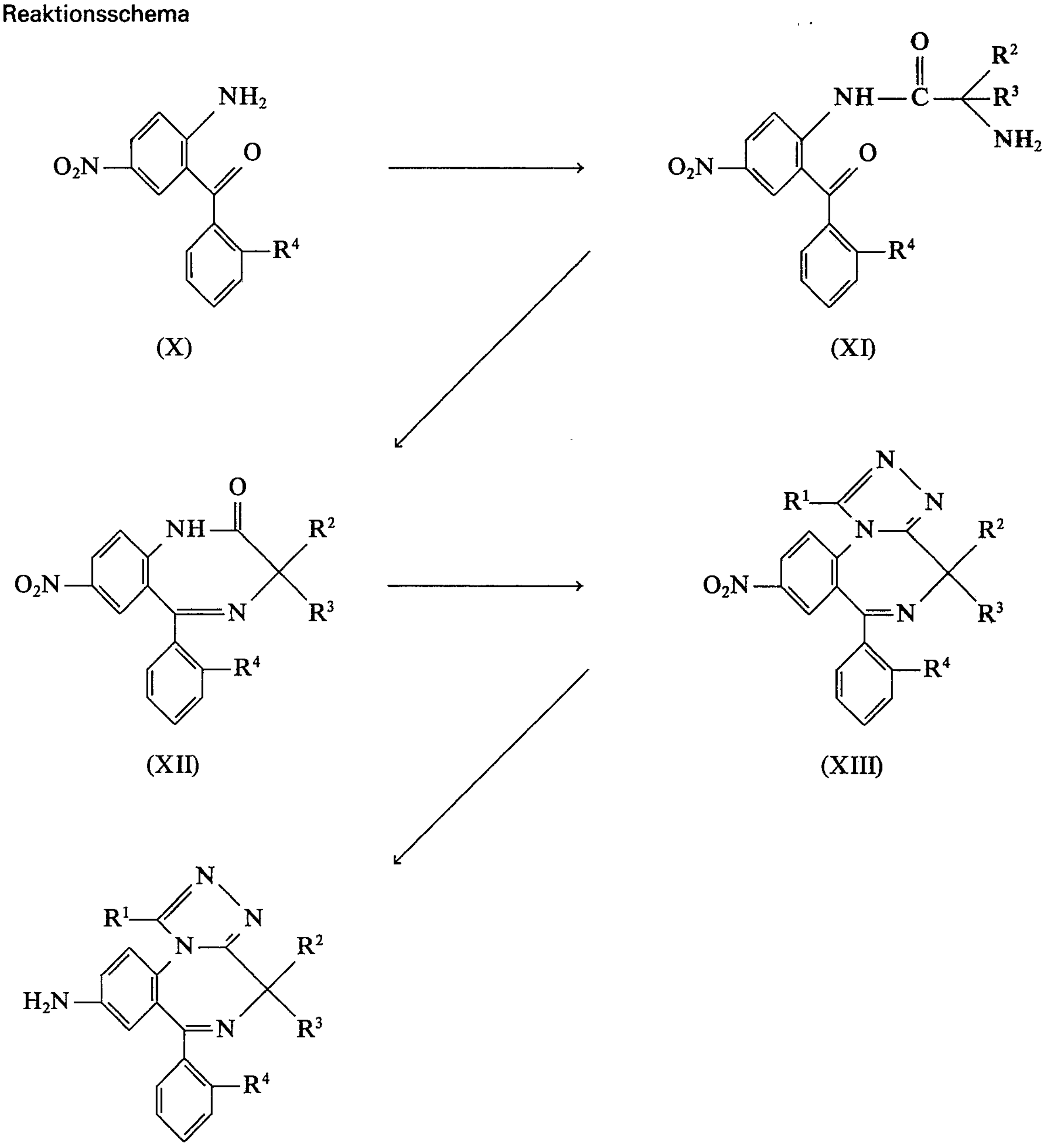

(X) (XI) (XII) (XIII) (IV)

Verschiedene der weiter unten folgenden Beispiele enthalten detaillierte Angaben betreffend die Herstellung bestimmter Verbindungen der allgemeinen Formel IV.

Verbindungen der allgemeinen Formel VII können nach verschiedenen, an sich bekannten Methoden aus Verbindungen der Formeln II oder IV hergestellt werden, wobei natürlich die Natur der Schutzgruppe bzw. Schutzgruppen, deren Anwesenheit in der herzustellenden Verbindung der Formel VII erwünscht ist, für die Wahl der zur Anwendung gelangenden Methode bzw. Methoden in Betracht gezogen werden muß.

Zur Herstellung einer Verbindung der Formel VII, worin $R^{53}$ eine Schutzgruppe bedeutet, kann man ein Benzodiazepin-Derivat der Formel IV mit einem entsprechenden Carbamoylhalogenid umsetzen (in Analogie zu der weiter oben beschriebenen Methode für die Herstellung von Verbindungen der Formel I aus Verbindungen der Formeln IV und V).

Zur Herstellung von Verbindung der Formel VII, worin $R^{53}$ Wasserstoff und $R^{63}$ einen Rest der Formel VIII bedeuten, kann man ein Benzodiazepin-Derivat der allgemeinen Formel IV mit einem entsprechenden Isocyanat umsetzen ( (in Analogie zur weiter oben beschriebenen Methode für die Herstellung von Verbindungen der Formel I aus Verbindungen der Formeln IV und VI). Eine andere Möglichkeit besteht in der Umsetzung eines Benzodiazepin-Derivats der allgemeinen Formel II mit einem entsprechenden Amin (in Analogie zu der weiter oben beschriebenen Methode für die Herstellung von Verbindungen der Formel I aus Verbindungen der Formeln II und III); dabei ist jedoch

zu beachten, daß in diesem Fall als Schutzgruppe (Y) eine Acylgruppe nicht in Betracht kommt. Weiterhin kann man in einem Benzodiazepin-Derivat der Formel Ia den Aziridinring durch saure Alkoholyse mit tert.-Butanol, Benzylalkohol o. dgl. öffnen, wobei man eine Verbindung der Formel VII erhält, worin $R^{53}$ Wasserstoff und $R^{63}$ 2-tert.-Butoxyäthyl bzw. 2-Benzyloxyäthyl bedeuten.

Zur Herstellung von Verbindungen der Formel VII, worin $R^{53}$ niederes Alkyl und $R^{63}$ einen Rest der Formel VIII bedeuten, kann man ein Benzodiazepin der allgemeinen Formel IV mit einem entsprechenden Carbamoylhalogenid umsetzen (in Analogie zu der weiter oben beschriebenen Methode für die Herstellung von Verbindungen der Formel I aus Verbindungen der Formeln IV und V). Eine andere Möglichkeit besteht in der Umsetzung eines Benzodiazepin-Derivats der allgemeinen Formel II mit einem entsprechenden Amin (in Analogie zur weiter oben beschriebenen Methode für die Herstellung von Verbindungen der Formel I aus Verbindungen der Formeln II und III), wobei wiederum als Schutzgruppe (Y) eine Acylgruppe nicht in Betracht kommt.

Die Verbindungen der allgemeinen Formel VII sind ebenfalls Gegenstand der vorliegenden Erfindung.

Verbindungen der Formel IX können nach an sich bekannten Methoden aus Aminobenzodiazepin-Derivaten der allgemeinen Formel IV hergestellt werden und zwar durch Umsetzen mit einem entsprechenden Carbamoylchlorid (in Analogie zur weiter oben beschriebenen Methode für die Herstellung von Verbindungen der Formel I aus Verbindungen der Formeln IV und V), wenn $R^{54}$ niederes Alkyl bedeutet, oder durch Umsetzung mit einem entsprechenden Isocyanat (in Analogie zur weiter oben beschriebenen Methode für die Herstellung von Verbindungen der Formel I aus Verbindungen der Formeln IV und VI), wenn $R^{54}$ Wasserstoff bedeutet.

Die Verbindungen der Formel IX sind ebenfalls Gegenstand der vorliegenden Erfindung.

Überraschenderweise hat es sich gezeigt, daß die Benzodiazepin-Derivate der eingangs definierten allgemeinen Formel I keine oder nur sehr geringe Wirkungen auf das zentrale Nervensystem entfalten, wogegen sie ausgeprägte aldosteronantagonistische Eigenschaften aufweisen. Diese aldosteronantagonistischen Eigenschaften lassen sich, wie nachstehend erläutert, an adrenalektomierten Ratten nachweisen.

Verabreicht man adrenalektomierten Ratten Aldosteron, so beobachtet man — im Vergleich zu unbehandelten Tieren — eine ausgeprägte Verminderung der Natrium-Ausscheidung (Natrium-Retention), eine erhöhte Kalium-Ausscheidung (Kalium-Exkretion), sowie eine Verminderung des ausgeschiedenen Harnvolumens. Gibt man den Tieren vor der Behandlung mit Aldosteron Verbindungen der Formel I, so beobachtet man — im Vergleich zu nur mit Aldosteron behandelten Tieren (Kontrolltiere) — eine ausgeprägte Erhöhung der Natrium-Ausscheidung (das heißt: die durch Aldosteron bewirkte Natrium-Retention wird antagonisiert), wogegen die Kalium-Exkretion und das Harnvolumen in geringerem Ausmaß beeinflußt werden.

Der Standardversuch wird wie folgt durchgeführt:

Weibliche Holtzmann-Ratten (150—180 g) werden 70 bis 74 Stunden vor Versuchsbeginn bilateral adrenalektomiert. Nach der Operation erhalten die Tiere ein gebräuchliches Ratten-Trockenfutter und 0,9proz. Kochsalzlösung zum Trinken. 16 bis 17 Stunden vor Versuchsbeginn wird den Tieren das Futter entzogen, wogegen sie nach wie vor ad libitum 0,9proz. Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subcutane Injektion von 4 mmg/kg Aldosteron. Nach weiteren 90 Minuten werden die Harnblasen der Tiere durch sorgfältiges suprapubisches Drücken entleert, worauf die Tiere ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluß des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertetern der durch die allgemeine Formel I definierten Verbindungsklasse in dem vorstehend geschilderten Versuch erhalten worden sind. Angegeben werden in dieser Tabelle für jede der darin figurierenden Verbindungen die verabreichte Dosis (in mg/kg p. o.) sowie die prozentuale Veränderung des Harnvolumens, der Natrium-Ausscheidung und der Kalium-Ausscheidung im Vergleich zu den Kontrollieren (das heißt im Vergleich zu den nur mit Aldosteron behandelten Tieren). Außerdem enthält die Tabelle Angaben über die akute Toxizität der untersuchten Verbindungen (DL 50 in mg/kg bei einmaliger oraler Verabreichung an Mäuse).

| $R_1$ | $R_2$ | $R_3$ | $R_4$ | $R_5$ | $R_6$ | Dosis mg/kg/p. o. | Volumen | $[Na^{\oplus}]$ | $[K^{\oplus}]$ | DL 50 mg/kg/p. o. |
|---|---|---|---|---|---|---|---|---|---|---|
| | | | | | | | in % bez. auf Kontrolltiere | | | |
| H | $CH_3$ | $CH_3$ | F | $CH_3$ | $CH_3$ | 1 | 106 | 238 | 65 | >5000 |
| $CH_3$ | $CH_3$ | $CH_3$ | F | H | $CH_2CH_2OH$ | 1 | 112 | 256 | 90 | >5000 |
| H | H | H | Cl | $CH_3$ | $CH_3$ | 10 | 131 | 252 | 100 | >5000 |
| $CH_3$ | H | H | Cl | $CH_3$ | $CH_3$ | 10 | 103 | 252 | 112 | >5000 |
| H | $CH_3$ | $CH_3$ | Cl | $-CH_2-CH_2-CH_2-CH_2-$ | | 1 | 97 | 155 | 72 | >5000 |
| $CH_3$ | $CH_3$ | $CH_3$ | Cl | H | $CH_2CH_2OH$ | 1 | 75 | 215 | 75 | >5000 |

Die Benzodiazepin-Derivate der allgemeinen Formel I und ihre pharmazeutisch akzeptablen Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Benzodiazepin-Derivate der allgemeinen Formel I und ihre pharmazeutisch akzeptablen Säureadditionssalze mit pharmazeutisch inerten, anorganischen oder organischen Excipienten verarbeitet werden. Als solche Excipientien kann man für Tabletten, Dragées und Hartgelatinekapseln z. B. Lactose, Maisstärke oder Derivate davon, Talk, Stearinsäure oder deren Salze verwenden.

Für Weichgelatinekapseln eignen sich als Excipientien z. B. pflanzliche Öle, Wachse, Fette, halbfeste und flüssige Polyole; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z. B. Wasser, Polyole, Saccharose, Invertzucker, Glukose.

Für Injektionslösungen eignen sich als Excipientien z. B. Wasser, Alkohole, Polyole, Glyzerin, pflanzliche Öle.

Für Suppositorien eignen sich als Excipientien z. B. natürliche oder gehärtete Öle, Wachse, Fette, halbflüssige oder flüssige Polyole.

Die pharmazeutischen Präparate können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend eine oder mehrere Verbindungen der allgemeinen Formel I oder pharmazeutisch akzeptable Säureadditionssalze davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch akzeptable Säureadditionssalze davon und gegebenenfalls einen oder mehrere andere therapeutisch wertvolle Stoffe in eine galenische Darreichungsform bringt. Die Benzodiazepin-Derivate der allgemeinen Formel I und pharmazeutisch akzeptable Säureadditionssalze davon können bei der Bekämpfung bzw. Verhütung von Krankheiten, insbesondere bei der Bekämpfung bzw. Verhütung von Herzversagen, von hepatitischer Aszites, von primärem Aldosteronismus und von essentieller Hypertonie verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von 20 mg bis 1500 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) 6 g (20,5 mMol) 8-Amino-6-(o-fluorophenyl)-4H-s-triazolo[4,3-a] [1,4]benzodiazepin werden in 600 ml 1,2-Dichloräthan unter Rückfluß in Lösung gebracht. In einem Sulfierkolben werden 3,3 g (33,3 mMol) Phosgen, gelöst in 50 ml eisgekühltem 1,2-Dichloräthan, vorgelegt. Die heiße Dichloräthanlösung des 8-Amino-6-(o-fluorophenyl)-4H-s-triazolo[4,3-a] [1,4]benzodiazepins wird dann unter Eiskühlung und Rühren derart zugetropft, daß die Reaktionstemperatur 20° nicht übersteigt. Anschließend wird das Reaktionsgemisch 1 Stunde unter Rühren am Rückfluß erhitzt, darauf 100 ml Dichloräthan abdestilliert und dann mit 100 ml frischem Dichloräthan versetzt. Unter Eiskühlung wird Argon direkt in die Reaktionslösung eingeleitet, bis diese eine Temperatur von 10° erreicht. Die so erhaltene Dichloräthanlösung von [6-(o-Fluorphenyl)-4H-s-triazolo[4,3-a] [1,4]benzodiazepin-8-yl]isocyanat wird ohne Isolierung des darin enthaltenen Isocyanates weiterverarbeitet.

b) Eine Suspension von 7,8 g (73,6 mMol) Natriumcarbonat in 3,7 ml (61,6 mMol) Äthanolamin und 60 ml Acetonitril wird auf einmal unter Eiskühlung und Rühren in die unter a) beschriebene Isocyanatlösung gegeben, worauf 20 Stunden bei Raumtemperatur gerührt, dann am Rotationsverdampfer bei 50° eingeengt wird.

Der Rückstand wird mit 500 ml Wasser versetzt und mehrmals mit einem 4 : 1-Gemisch von Methylenchlorid und Äthanol extrahiert, worauf die vereinigten organischen Auszüge mit Magnesiumsulfat getrocknet und eingeengt werden.

Kristallisieren aus Äthanol liefert 1-[6-(o-Fluorphenyl)-4H-s-triazolo[4,3-a] [1,4]benzodiazepin-8-yl]-3-(2-hydroxyäthyl)-harnstoff vom Smp. 155° (Zersetzung).

Beispiel 2

a) Man stellt eine Dichloräthanlösung von [6-(o-Fluorphenyl)-4H-s-triazolo[4,3-a] [1,4]benzodiazepin-8-yl]isocyanat, gemäß Angaben in Absatz a) von Beispiel 1 aus 5 g (17,05 mMol) 8-Amino-6-(o-fluorphenyl)-4H-s-triazolo[4,3-a] [1,4]benzodiazepin her.

b) Zu dieser Isocyanatlösung wird eine Suspension von 6,5 g (61,3 mMol) Natriumcarbonat in 5,2 g (115,6 mMol) Dimethylamin und 50 ml eisgekühltem Dichloräthan auf einmal unter Eiskühlen und Rühren gegeben. Nach 20 Stunden bei Raumtemperatur wird im Vakuum bei 50° eingeengt, der Rückstand in 300 ml Wasser suspendiert und 4mal mit Methylenchlorid/Äthanol (4 : 1) ausgeschüttelt. Die vereinigten organischen Auszüge werden mit Wasser gewaschen, getrocknet und eingeengt. Aus Methylenchlorid/Essigester kristallisiert 3-[6-(o-Fluorphenyl)-4H-s-triazolo[4,3-a] [1,4]benzodiazepin-8-yl]-1,1-dimethylharnstoff vom Smp. 206° (Zersetzung).

Beispiel 3

Eine Dichloräthanlösung von [6-(o-Fluorphenyl)-1-methyl-4H-s-triazolo[4,3-a] [1,4]benzodiazepin-8-yl]isocyanat, erhalten gemäß Angaben in Absatz a) von Beispiel 1 aus 5 g (16,3 mMol) 8-Amino-6-(o-fluorphenyl)-1-methyl-4H-s-triazolo[4,3-a] [1,4]benzodiazepin, wird zu einer Suspension von 6,2 g (58,5 mMol) Natriumcarbonat in 3 ml (49,8 mMol) Äthanolamin und 40 ml Acetonitril unter Rühren bei Raumtemperatur zugetropft. Nach 20 Stunden verteilt man zwischen 500 ml Wasser und 500 ml eines 4 : 1-Gemisches aus Methylenchlorid und Äthanol bei 50°. Die abgetrennte wäßrige Phase wird nochmals analog behandelt, die vereinigten organischen Extrakte werden getrocknet und im Vakuum eingeengt.

Kristallisation des Rückstandes aus Äthanol liefert 1-[6-(o-Fluorphenyl)-1-methyl-4H-s-triazolo-[4,3-a] [1,4]benzodiazepin-8-yl]-3-(2-hydroxyäthyl)harnstoff vom Smp. 228—230°.

Beispiel 4

a) 42,3 g (0,41 Mol) α-Amino-isobuttersäure und 50 ml (0,68 Mol) Thionylchlorid in 400 ml Tetrahydrofuran werden während 24 Stunden bei Raumtemperatur kräftig gerührt, mit 100 g (0,38 Mol) 2-Amino-5-nitro-2′-fluorbenzophenon versetzt und während weiteren 108 Stunden bei Raumtemperatur gerührt.

Das Reaktionsgemisch wird im Vakuum eingeengt, auf 500 ml eisgekühlte 10proz. Natriumbicarbonatlösung gegossen und mit Methylenchlorid extrahiert. Nach Waschen der organischen Phase mit Natriumbicarbonatlösung und Wasser wird das Lösungsmittel im Vakuum entfernt und das verbleibende, rohe 2-Amino-2′-(o-fluorbenzoyl)-2-methyl-4′-nitropropionanilid direkt weiterverarbeitet.

b) 184 g des obigen Rohproduktes in 1 l Toluol und 100 ml Eisessig werden während 21 Stunden in einem Wasserabscheider unter Rückfluß zum Sieden erhitzt und anschließend im Vakuum zur Trockne eingedampft. Der Rückstand wird in 500 ml siedendem Äther aufgeschlämmt und abfiltriert. Es resultiert 5-(o-Fluorphenyl)-1,3-dihydro-3,3-dimethyl-7-nitro-2H-1,4-benzodiazepin-2-on vom Smp. 241°.

c) 55,25 g (0,16 Mol) 5-(o-Fluorphenyl)-1,3-dihydro-3,3-dimethyl-7-nitro-2H-1,4-benzodiazepin-2-on, gelöst in 800 ml Tetrahydrofuran, versetzt man portionsweise und unter Rühren bei Raumtemperatur mit 9,7 g 55 bis 60proz. Natriumhydriddispersion und rührt noch 60 Minuten bei 50°. Zur abgekühlten Lösung fügt man 100 g (0,39 Mol) Dimorpholinochlorphosphat zu und rührt 24 Stunden bei Raumtemperatur. Nach Zugabe von 50 g (0,67 Mol) Essigsäurehydrazid rührt man das Gemisch weitere 45 Stunden bei Raumtemperatur, engt im Vakuum ein, nimmt den Rückstand in Methylenchlorid auf, wäscht die organische Phase mit Wasser und entfernt das Lösungsmittel. Der Rückstand wird mit 1,2 l n-Butanol versetzt, und unter Rückfluß zum Sieden erhitzt. Nach Abdestillieren von 350 ml Lösungsmittel erhitzt man noch 1 Stunde am Rückfluß und engt im Vakuum ein. Der Rückstand wird in 250 ml heißem Äthanol aufgelöst, nach Abkühlen auf −10° erhält man kristallines 6-(o-Fluorphenyl)-1,4,4-trimethyl-8-nitro-4H-s-triazolo[4,3-a] [1,4]benzodiazepin vom Smp. 195°.

d) 43,6 g (0,12 Mol) 6-(o-Fluorphenyl)-1,4,4-trimethyl-8-nitro-4H-s-triazolo[4,3-a] [1,4]benzodiazepin in 600 ml konz. Salzsäure werden portionsweise mit insgesamt 80,55 g (0,36 Mol) Zinnchlorid versetzt, so daß die Temperatur des Reaktionsgemisches 85° nicht übersteigt. Anschließend rührt man 1 Stunde bei Raumtemperatur, kühlt auf 0° ab und neutralisiert vorsichtig mit 900 ml 10 N Natronlauge. Nach dreimaligem Extrahieren der verbleibenden Suspension mit Methylenchlorid werden die vereinigten organischen Phasen mit gesättigter Natriumchloridlösung gewaschen, getrocknet und eingedampft. Umkristallisieren des Rückstandes aus Äthanol (350 ml, −30°) 8-Amino-6-(o-fluorphenyl)-1,4,4-trimethyl-4H-s-triazolo[4,3-a] [1,4]benzodiazepin vom Smp. 294°.

e) Aus 7,0 g (20,9 mMol) 8-Amino-6-(o-fluorphenyl)-1,4,4-trimethyl-4H-s-triazolo[4,3-a] [1,4]benzodiazepin wird in Analogie zur Arbeitsvorschrift von Beispiel 1, über das [6-(o-Fluorphenyl)-1,4,4-trimethyl-4H-s-triazolo[4,3-a] [1,4]benzodiazepin-8-yl]isocyanat, 1-[6-(o-Fluorphenyl)-1,4,4-trimethyl-4H-s-triazolo[4,3-a] [1,4]benzodiazepin-8-yl]-3-(2-hydroxyäthyl)harnstoff erhalten. Nach Umkristallisieren aus Acetonitril schmilzt das Produkt bei 233°.

## Beispiel 5

a) Zu einer Lösung von 49,5 g (0,15 Mol) 5-(o-Fluorphenyl)-1,3-dihydro-3,3-dimethyl-7-nitro-2H-1,4-benzodiazepin-2-on (hergestellt gemäß den Angaben von Absatz a) und b) in Beispiel 4) in 800 ml Tetrahydrofuran gibt man portionsweise und unter Rühren bei Raumtemperatur 8,75 g 55 bis 60proz. Natriumhydriddispersion (0,36 M) zu und rührt das Gemisch noch 1 Stunde bei 50° unter Argon. 96,6 g (0,39 Mol) Dimorpholinochlorophosphat werden nach dem Abkühlen auf Raumtemperatur hinzugefügt, worauf das Reaktionsgemisch während 20 Stunden bei derselben Temperatur gerührt wird. Nach Zugabe von 48,5 g (0,80 M) Formylhydrazin rührt man während 24 Stunden bei Raumtemperatur unter Argon, engt im Vakuum ein und nimmt den Rückstand in Methylenchlorid auf. Die organische Phase wird mit Wasser gewaschen und getrocknet. Nach Entfernen des Lösungsmittels wird der Rückstand in 1 l n-Butanol aufgenommen und zum Sieden erhitzt. Nach Abdestillieren von 400 ml Lösungsmittel erhitzt man noch 1 Stunde am Rückfluß, entfernt das Lösungsmittel im Vakuum und filtriert den Rückstand über 800 g Silicagel unter Eluieren mit Essigester/Äthanol (9 : 1). Das so gewonnene Rohprodukt ergibt nach Imkristallisieren aus Äthanol 6-(o-Fluorphenyl)-4,4-dimethyl-8-nitro-4H-s-triazolo[4,3-a] [1,4]benzodiazepin vom smp. 235°.

b) 22,6 g (64,5 mMol) 6-(o-Fluorphenyl)-4,4-dimethyl-8-nitro-4H-s-triazolo[4,3-a] [1,4]benzodiazepin, gelöst in 200 ml konz. Salzsäure, werden portionsweise mit insgesamt 43,66 g (0,19 Mol) Zinnchlorid versetzt, so daß die Reaktionstemperatur 85° nicht übersteigt. Man rührt noch 1 Stunde bei Raumtemperatur, neutralisiert vorsichtig unter Eiskühlung mit 10 N Natronlauge und extrahiert in einem Perforator mit Chloroform/Äthanol (9 : 1).

Nach dem Entfernen des Lösungsmittels wird der Rückstand in heißem Essigester aufgeschlämmt und abfiltriert, wobei 8-Amino-6-(o-fluorphenyl)-4,4-dimethyl-4H-s-triazolo[4,3-a] [1,4]benzodiazepin vom Smp. 288° resultiert.

c) Aus einer Dichloräthanlösung von [6-(o-Fluorphenyl)-4,4-dimethyl-4H-s-triazolo[4,3-a] [1,4]benzodiazepin-8-yl]isocyanat, erhalten gemäß Angaben in Absatz a) von Beispiel 1 aus 7,0 g (21,8 mMol) 8-Amino-6-(o-fluorphenyl)-4,4-dimethyl-4H-s-triazolo[4,3-a] [1,4]benzodiazepin, wird in Analogie zur Arbeitsvorschrift in Absatz b) von Beispiel 2 3-[6-(o-Fluorphenyl)-4,4-dimethyl-4H-s-triazolo-[4,3-a] [1,4]benzodiazepin-8-yl]-1,1-dimethylharnstoff erhalten. Durch Umkristallisieren aus kaltem Methylenchlorid erhält man ein Produkt vom Smp. 180° (Zersetzung).

## Beispiel 6

Aus einer Dichloräthanlösung von [6-(o-Fluorphenyl)-1,4,4-trimethyl-4H-s-triazolo[4,3-a] [1,4]benzodiazepin-8-yl]isocyanat, erhalten gemäß Angaben in Absatz a) von Beispiel 1 aus 7,0 g (20,9 mMol) 8-Amino-6-(o-fluorphenyl)-1,4,4-trimethyl-4H-s-triazolo[4,3-a] [1,4]benzodiazepin, wird in Analogie zur Arbeitsvorschrift in Absatz b) von Beispiel 2 3-[6-(o-Fluorphenyl)-1,4,4-trimethyl-4H-s-triazolo-[4,3-a] [1,4]benzodiazepin-8-yl]-1,1-dimethylharnstoff erhalten. Aus Methylenchlorid/Toluol umkristallisiert, schmilzt die Substanz bei 285—286°.

## Beispiel 7

a) Man stellt eine Lösung von [6-(o-Chlorphenyl)-4H-s-triazolo[4,3-a] [1,4]benzodiazepin-8-yl]isocyanat, gemäß Angaben in Absatz a) von Beispiel 1 aus 7,0 g (22,6 mMol) 8-Amino-6-(o-chlorphenyl)-4H-s-triazolo[4,3-a] [1,4]benzodiazepin, jedoch unter Verwendung von Methylenchlorid anstelle von 1,2-Dichloräthan, her.

b) Zu dieser Isocyanatlösung wird eine Suspension von 8,7 g (81,9 mMol) Natriumcarbonat in 4,9 ml (67,8 mMol) Äthanolamin und 60 ml Acetonitril auf einmal und unter Rühren bei Raumtemperatur gegeben. Nach 20 Stunden bei Raumtemperatur wird im Vakuum bei 50° eingeengt, der Rückstand in Methylenchlorid/Äthanol (4 : 1) aufgenommen, die organische Phase mit Wasser gewaschen, getrocknet und eingeengt. Durch Kristallisieren aus Äthanol erhält man 1-[6-(o-Chlorphenyl)-4H-s-triazolo[4,3-a] [1,4]benzodiazepin-8-yl]-3-(2-hydroxyäthyl)harnstoff vom Smp. 228° (Zersetzung).

## Beispiel 8

Zu einer Lösung von [6-(o-Chlorphenyl)-4H-s-triazolo[4,3-a] [1,4]benzodiazepin-8-yl]isocyanat, erhalten gemäß Angaben in Absatz a) von Beispiel 1 aus 7,0 g (22,6 mMol) 8-Amino-6-(o-chlorphenyl)-4H-s-triazolo[4,3-a] [1,4]benzodiazepin, jedoch unter Verwendung von Methylenchlorid anstelle von 1,2-Dichloräthan, wird eine Suspension von 8,7 g (81,9 mMol) Natriumcarbonat in 5,3 g (117,8 mMol) Dimethylamin und 50 ml 1,2-Dichloräthan unter Eiskühlung und Rühren in einer Portion zugegeben. Nach 25 Stunden bei Raumtemperatur wird im Vakuum bei 50° eingedampft, der Rückstand in 300 ml Wasser suspendiert, 4mal mit Methylenchlorid/Äthanol (4 : 1) extrahiert und die vereinigten organischen Auszüge anschließend getrocknet und eingedampft.

Aus Methylenchlorid/Essigester kristallisiert 3-[6-(o-Chlorphenyl)-4H-s-triazolo[4,3-a] [1,4]benzodiazepin-8-yl]-1,1-dimethylharnstoff vom Smp. 180° (Zersetzung).

## Beispiel 9

Zu einer Lösung von [6-(2-Chlorphenyl)-1-methyl-4H-s-triazolo[4,3-a] [1,4]benzodiazepin-8-yl]isocyanat, erhalten gemäß Angaben in Absatz a) von Beispiel 1 aus 7,0 g (21,6 mMol) 8-Amino-6-(2-chlorphenyl)-1-methyl-4H-s-triazolo[4,3-a] [1,4]benzodiazepin, jedoch unter Verwendung von Methylenchlorid anstelle von 1,2-Dichloräthan, wird eine Suspension von 8,7 g (81,9 mMol) Natriumcarbonat in 4,2 ml (35,4 mMol) Äthanolamin und 60 ml Acetonitril unter Rühren und Eiskühlung in einer Portion zugegeben. Nach 27 Stunden bei Raumtemperatur wird im Vakuum bei 50° eingeengt, der Rückstand in Methylenchlorid suspendiert, das feste Material abgenutscht und aus Äthanol umkristallisiert. Dabei erhält man 1-[6-(o-Chlorphenyl)-1-methyl-4H-s-triazolo[4,3-a] [1,4]benzodiazepin-8-yl]-3-(2-hydroxyäthyl)harnstoff vom Smp. 188° (Zersetzung).

## Beispiel 10

Eine Lösung von [6-(2-Chlorphenyl)-1-methyl-4H-s-triazolo[4,3-a] [1,4]benzodiazepin-8-yl]isocyanat, erhalten gemäß Angaben in Absatz a) von Beispiel 1 aus 7,0 g (21,6 mMol) 8-Amino-6-(2-chlorphenyl)-1-methyl-4H-s-triazolo[4,3-a] [1,4]benzodiazepin, jedoch unter Verwendung von Methylenchlorid anstelle von 1,2-Dichloräthan, wird unter Rühren und Eiskühlung mit einer Suspension von 8,7 g (81,9 mMol) Natriumcarbonat in 50 ml eisgekühltem Methylenchlorid und 5 g (111,1 mMol) Dimethylamin auf einmal versetzt. Nach Zugabe von 60 ml trockenem Acetonitril wird das Reaktionsgemisch 20 Stunden bei Raumtemperatur gerührt, im Vakuum bei 50° eingeengt, der Rückstand in 100 ml Methylenchlorid und 100 ml Wasser aufgeschlämmt und abfiltriert. Das zurückbleibende Material wird in 80 ml heißem Äthanol gelöst und über Nacht bei −10° stehen gelassen. Durch Abfiltrieren der erhaltenen Kristalle erhält man 3-[6-(o-Chlorphenyl)-1-methyl-4H-s-triazolo[4,3-a] [1,4]benzodiazepin-8-yl]-1,1-dimethylharnstoff vom Smp. 186° (Zersetzung).

## Beispiel 11

a) Man stellt eine Lösung von [6-(2-Chlorphenyl)-1-methyl-4H-s-triazolo[4,3-a] [1,4]benzodiazepin-8-yl]isocyanat, gemäß Angaben in Absatz a) von Beispiel 1 aus 7,0 g (21,6 mMol) 8-Amino-6-(2-chlorphenyl)-1-methyl-4H-s-triazolo[4,3-a] [1,4]benzodiazepin, jedoch unter Verwendung von Methylenchlorid an Stelle von 1,2-Dichloräthan, her.

b) diese Isocyanatlösung wird unter Rühren und Eiskühlung mit einer Suspension von 8,7 g (81,9 mMol) Natriumcarbonat in 5,9 ml (70 mMol) Pyrrolidin und 60 ml Acetonitril versetzt. Das Gemisch wird 68 Stunden bei Raumtemperatur gerührt im Vakuum bei 50° eingeengt und der Rückstand 2mal mit je 500 ml Methylenchlorid/Äthanol (4 : 1) extrahiert. Die organischen Phasen werden mit 3 N Salzsäure und Wasser gewaschen, getrocknet und eingedampft. Der Rückstand, aus 400 ml Methylenchlorid umkristallisiert, ergibt N-[6-(o-Chlorphenyl)-1-methyl-4H-s-triazolo-[4,3-a] [1,4]benzodiazepin-8-yl]-1-pyrrolidin carboxamid, das unter Zersetzung bei 188° schmilzt.

## Beispiel 12

a) Aus 55 g (0,2 mMol) 2-Amino-5-nitro-2′-chlorbenzophenon erhält man in Analogie zu den Arbeitsvorschriften in Absatz a) und b) von Beispiel 4 5-(o-Chlorphenyl)-1,3-dihydro-3,3-dimethyl-7-nitro-2H-1,4-benzodiazepin-2-on, das aus Methylenchlorid/Hexan umkristallisiert, bei 242° schmilzt.

b) In Analogie zur Arbeitsvorschrift in Absatz c) von Beispiel 4 wird aus 20 g (58 mMol) 5-(o-Chlorphenyl)-1,3-dihydro-3,3-dimethyl-7-nitro-2H-1,4-benzodiazepin-2-on 6-(o-Chlorphenyl)-1,4,4-trimethyl-8-nitro-4H-s-triazolo[4,3-a] [1,4]benzodiazepin erhalten, das aus Äthanol umkristallisiert, bei 283° schmilzt.

c) In Analogie zur Arbeitsvorschrift in Absatz d) von Beispiel 4 wird aus 48,5 g (0,127 Mol) des obigen, in Absatz b) beschriebenen Produktes 8-Amino-6-(o-chlorphenyl)-1,4,4-trimethyl-4H-s-triazolo[4,3-a] [1,4]benzodiazepin erhalten. Nach Umkristallisieren aus Äthanol schmilzt das Produkt bei 289°.

d) Aus einer Lösung von [6-(o-Chlorphenyl)-1,4,4-trimethyl-4H-s-triazolo[4,3-a] [1,4]benzodiazepin-8-yl]isocyanat, erhalten gemäß Angaben in Absatz a) von Beispiel 1 aus 7 g (19,9 mMol) 8-Amino-6-(o-chlorphenyl)-1,4,4-trimethyl-4H-s-triazolo[4,3-a] [1,4]benzodiazepin, jedoch unter Verwendung von Methylenchlorid anstelle von 1,2-Dichloräthan, erhält man in Analogie zur Arbeitsvorschrift in Absatz b) von Beispiel 7 1-[6-(o-Chlorphenyl)-1,4,4-trimethyl-4H-s-triazolo-[4,3-a] [1,4]benzodiazepin-8-yl)-3-(2-hydroxyäthyl)harnstoff, der nach Umkristallisieren aus Toluol/Äthanol bei 218 – 220° schmilzt.

### Beispiel 13

a) In Analogie zur Arbeitsvorschrift in Absatz a) von Beispiel 5 wird aus 53,6 g (0,15 Mol) 5-(o-Chlorphenyl)-1,3-dihydro-3,3-dimethyl-7-nitro-2H-1,4-benzodiazepin-2-on (Beispiel 12, Absatz a), 6-(o-Chlorphenyl)-4,4-dimethyl-8-nitro-4H-s-triazolo[4,3-a] [1,4]benzodiazepin erhalten, das, aus Äthanol umkristallisiert, bei 262° schmilzt.

b) In Analogie zur Arbeitsvorschrift in Absatz b) von Beispiel 5 wird aus 21,2 g (57 mMol) des obigen, unter a) beschriebenen Produktes 8-Amino-6-(o-chlorphenyl)-4,4-dimethyl-4H-s-triazolo-[4,3-a] [1,4]benzodiazepin erhalten, das nach Umkristallisieren aus Essigester bei 264° schmilzt.

c) Aus einer Lösung von [6-(o-Chlorphenyl)-4,4-dimethyl-4H-s-triazolo[4,3-a] [1,4]benzodiazepin-8-yl]isocyanat, erhalten gemäß Angaben in Absatz a) von Beispiel 1 aus 7 g (20,7 mMol) des obigen unter b) beschriebenen Produktes, jedoch unter Verwendung von Methylenchlorid anstelle von 1,2-Dichloräthan, erhält man in Analogie zur Arbeitsvorschrift in Absatz b) von Beispiel 11 N-[6-(o-Chlorphenyl)-4,4-dimethyl-4H-s-triazolo[4,3-a] [1,4]benzodiazepin-8-yl]-1-pyrrolidincarboxamid, das nach Umkristallisieren aus Methylenchlorid bei 253° schmilzt.

### Beispiel 14

Aus 7,0 g (20,9 mMol) 8-Amino-6-(o-fluorphenyl)-1,4,4-trimethyl-4H-s-triazolo[4,3-a] [1,4]benzodiazepin wird, in Analogie zur Arbeitsvorschrift in Beispiel 1, über das [6-(o-Fluorphenyl)-1,4,4-trimethyl-4H-s-triazolo-[4,3-a] [1,4]benzodiazepin-8-yl]isocyanat, N-[6-(o-Fluorphenyl)-1,4,4-trimethyl-4H-s-triazolo[4,3-a] [1,4]benzodiazepin-8-yl]-1-pyrrolidincarboxamid erhalten. Aus Essigester/Äthanol umkristallisiert, schmilzt das Produkt bei 200° (Zersetzung).

### Beispiel A

1-[6-(o-Fluorphenyl)-1,4,4-trimethyl-4H-s-triazolo[4,3-a] [1,4]benzodiazepin-8-yl]-3-(2-hydroxyäthyl)-harnstoff kann wie folgt als Wirkstoff zur Herstellung von pharmazeutischen Präparaten verwendet werden:

a) Tabletten

1 Tablette enthält:

| | |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10proz. Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tablette von 12 mm Durchmesser mit Kreuzbruchrille verpreßt.

b) Kapseln

| | |
|---|---|
| 1 Kapsel enthält: | |
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Größe abgefüllt.

**Patentansprüche**

1. Benzodiazepin-Derivate der allgemeinen Formel

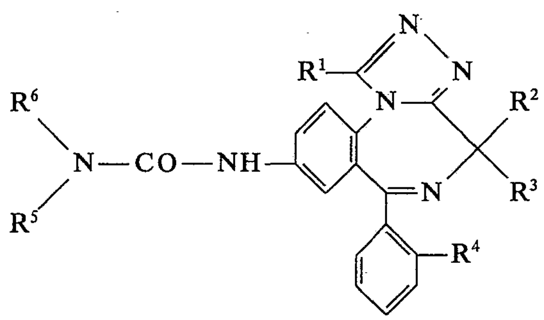

(I)

worin $R^1$ Wasserstoff oder $C_{1-7}$-Alkyl, $R^2$ und $R^3$ je Wasserstoff oder $C_{1-7}$-Alkyl und $R^4$ Halogen bedeuten und entweder $R^5$ Wasserstoff oder $C_{1-7}$-Alkyl und $R^6$ $C_{1-7}$-Alkyl oder $C_{2-7}$-Hydroxyalkyl bedeuten oder $R^5$ und $R^6$ zusammen mit dem Stickstoffatom einen 3- bis 7gliedrigen Heterocyclus bedeuten,
und pharmazeutisch akzeptable Säureadditionssalze von Verbindungen der Formel I.

2. Verbindungen gemäß Anspruch 1, dadurch gekennzeichnet, daß $R^1$ Wasserstoff oder Methyl bedeutet.

3. Verbindungen gemäß Anspruch 1 oder 2, dadurch gekennzeichnet, daß $R^2$ und $R^3$ beide Wasserstoff oder beide Methyl bedeuten.

4. Verbindungen gemäß einem der Ansprüche 1 bis 3, dadurch gekennzeichnet, daß $R^4$ Fluor oder Chlor bedeutet.

5. Verbindungen gemäß einem der Ansprüche 1 bis 4, dadurch gekennzeichnet, daß $R^5$ und $R^6$ beide Methyl oder $R^5$ Wasserstoff und $R^6$ Hydroxyäthyl oder $R^5$ und $R^6$ mit dem Stickstoffatom zusammen Pyrrolidinyl bedeuten.

6. 1-[6-(o-Fluorphenyl)-1,4,4-trimethyl-4H-s-triazolo[4,3-a][1,4]benzodiazepin-8-yl]-3-(2-hydroxyäthyl)harnstoff.

7. 1-[6-(o-Chlorphenyl)-1,4,4-trimethyl-4H-s-triazolo[4,3-a][1,4]benzodiazepin-8-yl]-3-(2-hydroxyäthyl)harnstoff.

8. 3-[6-(o-Fluorphenyl)-4,4-dimethyl-4H-s-triazolo[4,3-a][1,4]benzodiazepin-8-yl]-1,1-dimethyl)harnstoff.

9. 3-[6-(o-Chlorphenyl)-4H-s-triazolo[4,3-a][1,4]benzodiazepin-8-yl]-1,1-dimethylharnstoff.

10. 3-[6-(o-Chlorphenyl)-1-methyl-4H-s-triazolo[4,3-a][1,4]benzodiazepin-8-yl]-1,1-dimethylharnstoff.

11. N-[6-(o-Chlorphenyl)-4,4-dimethyl-4H-s-triazolo[4,3-a][1,4]benzodiazepin-8-yl]-1-pyrrolidincarboxamid.

12. 1-[6-(o-Fluorphenyl)-4H-s-triazolo[4,3-a][1,4]benzodiazepin-8-yl]-3-(2-hydroxyäthyl)harnstoff,
3-[6-(o-Fluorphenyl)-4H-s-triazolo[4,3-a][1,4]benzodiazepin-8-yl]-1,1-dimethylharnstoff,
1-[6-(o-Fluorphenyl)-1-methyl-4H-s-triazolo[4,3-a][1,4]benzodiazepin-8-yl]-3-(2-hydroxyäthyl)harnstoff,
3-[6-(o-Fluorphenyl)-1,4,4-trimethyl-4H-s-triazolo[4,3-a][1,4]benzodiazepin-8-yl]-1,1-dimethylharnstoff,
1-[6-(o-Chlorphenyl)-4H-s-triazolo[4,3-a][1,4]benzodiazepin-8-yl]-3-(2-hydroxyäthyl)harnstoff,

1-[6-(o-Chlorphenyl)-1-methyl-4H-s-triazolo[4,3-a][1,4]benzodiazepin-8-yl]-3-(2-hydroxyäthyl)-harnstoff,
N-[6-(o-Chlorphenyl)-1-methyl-4H-s-triazolo[4,3-a][1,4]benzodiazepin-8-yl]-1-Pyrrolidincarboxamid und
N-[6-(o-Fluorphenyl)-1,4,4-trimethyl-4H-s-triazolo[4,3-a][1,4]benzodiazepin-8-yl]-1-Pyrrolidincarboxamid.

13. Verbindungen der allgemeinen Formel

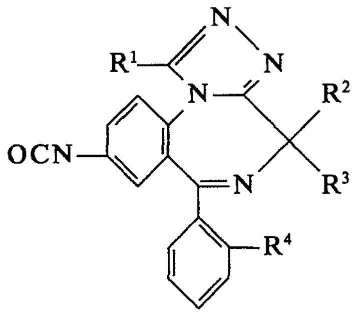

(II)

worin $R^1$, $R^2$, $R^3$ und $R^4$ die im Anspruch 1 angegebene Bedeutung besitzen.

14. Verbindungen der allgemeinen Formel

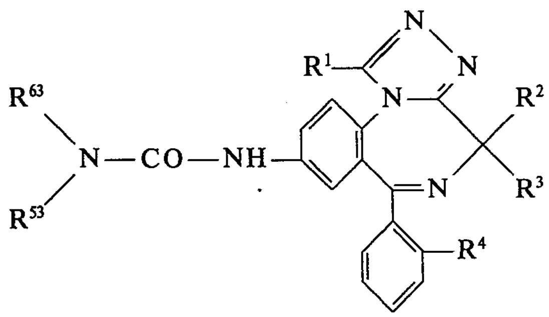

(VII)

worin $R^1$, $R^2$, $R^3$ und $R^4$ die im Anspruch 1 angegebene Bedeutung besitzen und entweder $R^{53}$ eine Schutzgruppe und $R^{63}$ $C_{1-7}$-Alkyl oder einen Rest der Formel

$$—A—O—Y \qquad (VIII)$$

bedeuten, wobei A $C_{2-7}$-Alkylen und Y eine Schutzgruppe bedeuten, oder $R^{53}$ Wasserstoff oder $C_{1-7}$-Alkyl und $R^{63}$ einen Rest der obigen Formel VIII bedeuten.

15. Verbindungen der allgemeinen Formel

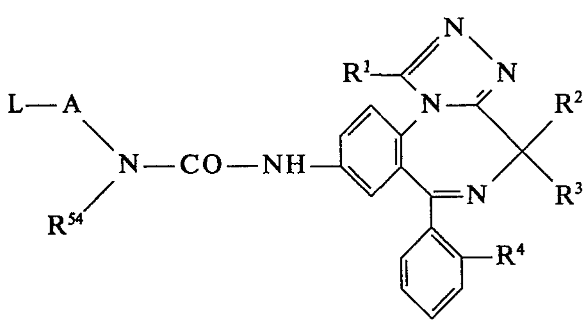

(IX)

worin $R^1$, $R^2$, $R^3$ und $R^4$ die in Anspruch 1 angegebene Bedeutung besitzen, und $R^{54}$ Wasserstoff oder $C_{1-7}$-Alkyl, L eine Abgangsgruppe und A $C_{2-7}$-Alkylen bedeuten.

16. Verbindungen gemäß einem der Ansprüche 1 bis 12 als pharmazeutische Wirkstoffe.

17. Verbindungen gemäß einem der Ansprüche 1 bis 12 als aldosteronantagonistische Wirkstoffe.

18. Verfahren zur Herstellung von Verbindungen gemäß einem der Ansprüche 1 bis 12, dadurch gekennzeichnet, daß man

a) ein Benzodiazepin-Derivat der allgemeinen Formel

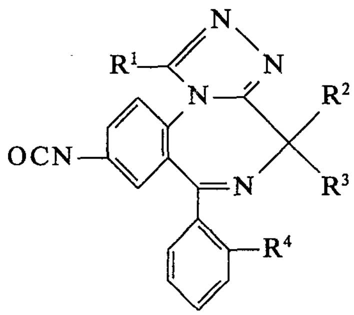

(II)

worin $R^1$, $R^2$, $R^3$ und $R^4$ die im Anspruch 1 angegebene Bedeutung besitzen, mit einer Aminoverbindung der allgemeinen Formel

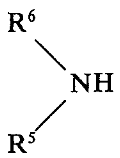

(III)

worin $R^5$ und $R^6$ die im Anspruch 1 angegebene Bedeutung besitzen, umsetzt, oder

b) ein Benzodiazepin-Derivat der allgemeinen Formel

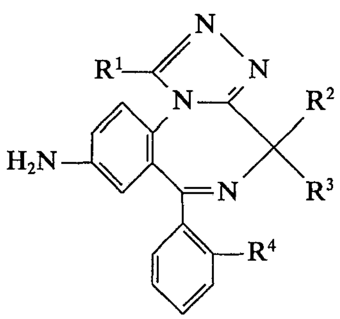

(IV)

worin $R^1$, $R^2$, $R^3$ und $R^4$ die im Anspruch 1 angegebene Bedeutung besitzen, mit einem Halogenid der allgemeinen Formel

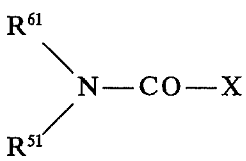

(V)

worin X Halogen bedeutet und entweder $R^{51}$ und $R^{61}$ je $C_{1-7}$-Alkyl bedeuten oder $R^{51}$ und $R^{61}$ zusammen mit dem Stickstoffatom einen 3- bis 7gliedrigen Heterocyclus bedeuten, umsetzt oder

c) ein Benzodiazepin-Derivat der obigen allgemeinen Formel IV mit einem Isocyanat der allgemeinen Formel

$R^{62}$—NCO (VI)

worin $R^{62}$ $C_{1-7}$-Alkyl bedeutet, umsetzt oder

d) aus einem Benzodiazepin-Derivat der allgemeinen Formel

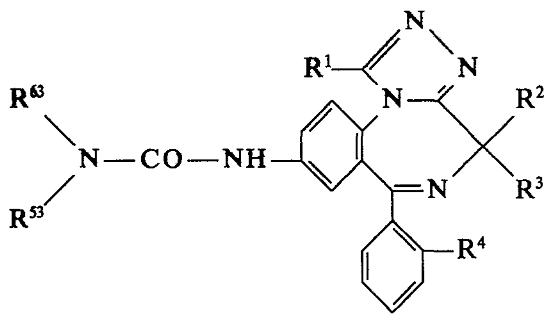

(VII)

worin $R^1$, $R^2$, $R^3$ und $R^4$ die im Anspruch 1 angegebene Bedeutung besitzen und entweder $R^{53}$ eine Schutzgruppe und $R^{63}$ $C_{1-7}$-Alkyl oder einen Rest der Formel

$$—A—O—Y \qquad \text{(VIII)}$$

bedeuten, wobei A $C_{2-7}$-Alkylen und Y eine Schutzgruppe bedeuten, oder $R^{53}$ Wasserstoff oder $C_{1-7}$-Alkyl und $R^{63}$ einen Rest der obigen Formel (VIII) bedeuten,
die Schutzgruppe(n) entfernt, oder

e) ein Benzodiazepin-Derivat der allgemeinen Formel

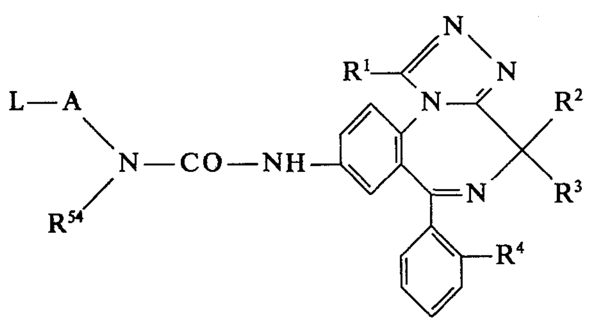

(IX)

worin $R^1$, $R^2$, $R^3$ und $R^4$ die im Anspruch 1 angegebene Bedeutung besitzen und $R^{54}$ Wasserstoff oder $C_{1-7}$-Alkyl und L eine Abgangsgruppe bedeuten,
in die entsprechende Hydroxyverbindung überführt, oder

f) in einem Benzodiazepin-Derivat der allgemeinen Formel

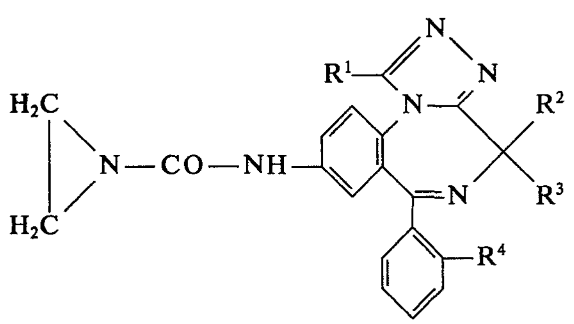

(Ia)

worin $R^1$, $R^2$, $R^3$ und $R^4$ die im Anspruch 1 angegebene Bedeutung besitzen,
den Aziridinring hydrolytisch öffnet, oder

g) ein Benzodiazepin-Derivat der im Anspruch 1 definierten allgemeinen Formel (I) in ein pharmazeutisch akzeptables Säureadditionssalz überführt.

19. Arzneimittel, enthaltend eine Verbindung gemäß einem der Ansprüche 1 bis 12 oder ein pharmazeutisch akzeptables Säureadditionssalz davon.

20. Aldosteron-antagonistische Arzneimittel, enthaltend eine Verbindung gemäß einem der Ansprüche 1 bis 12 oder ein pharmazeutisch akzeptables Säureadditionssalz davon.

## Claims

1. Benzodiazepine derivatives of the general formula

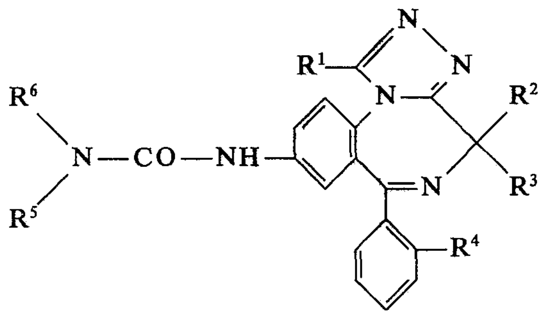

(I)

wherein $R^1$ signifies hydrogen or $C_{1-7}$-alkyl, $R^2$ and $R^3$ each signify hydrogen or $C_{1-7}$-alkyl and $R^4$ signifies halogen and either $R^5$ signifies hydrogen or $C_{1-7}$-alkyl and $R^6$ signifies $C_{1-7}$-alkyl or $C_{2-7}$-hydroxyalkyl or $R^5$ and $R^6$ together with the nitrogen atom signify a 3- to 7-membered heterocycle, and pharmaceutically acceptable acid addition salts of compounds of formula I.

2. Compounds according to claim 1, characterized in that $R^1$ signifies hydrogen or methyl.

3. Compounds according to claim 1 or 2, characterized in that $R^2$ and $R^3$ both signify hydrogen or both signify methyl.

4. Compounds according to any one of claims 1 to 3, characterized in that $R^4$ signifies fluorine or chlorine.

5. Compounds according to any one of claims 1 to 4, characterized in that $R^5$ and $R^6$ both signify methyl or $R^5$ signifies hydrogen and $R^6$ signifies hydroxyethyl or $R^5$ and $R^6$ with the nitrogen atom together signify pyrrolidinyl.

6. 1-[6-(o-Fluorophenyl)-1,4,4-trimethyl-4H-s-triazolo[4,3-a][1,4]benzodiazepin-8-yl]-3-(2-hydroxyethyl)urea.

7. 1-[6-(o-Chlorophenyl)-1,4,4-trimethyl-4H-s-triazolo[4,3-a][1,4]benzodiazepin-8-yl]-3-(2-hydroxyethyl)urea.

8. 3-[6-(o-Fluorophenyl)-4,4-dimethyl-4H-s-triazolo[4,3-a][1,4]benzodiazepin-8-yl]-1,1-dimethylurea.

9. 3-[6-(o-Chlorophenyl)-4H-s-triazolo[4,3-a][1,4]benzodiazepin-8-yl]-1,1-dimethylurea.

10. 3-[6-(o-Chlorophenyl)-1-methyl-4H-s-triazolo[4,3-a][1,4]benzodiazepin-8-yl]-1,1-dimethylurea.

11. N-[6-(o-Chlorophenyl)-4,4-dimethyl-4H-s-triazolo[4,3-a][1,4]benzodiazepin-8-yl]-1-pyrrolidinecarboxamide.

12. 1-[6-(o-Fluorophenyl)-4H-s-triazolo[4,3-a][1,4]benzodiazepin-8-yl]-3-(2-hydroxyethyl)-urea,
3-[6-(o-fluorophenyl)-4H-s-triazolo[4,3-a][1,4]benzodiazepin-8-yl]-1,1-dimethylurea,
1-[6-(o-fluorophenyl)-1-methyl-4H-s-triazolo[4,3-a][1,4]benzodiazepin-8-yl]-3-(2-hydroxyethyl)-urea,
3-[6-(o-fluorophenyl)-1,4,4-trimethyl-4H-s-triazolo[4,3-a][1,4]benzodiazepin-8-yl]-1,1-dimethylurea,
1-[6-(o-chlorophenyl)-4H-s-triazolo[4,3-a][1,4]benzodiazepin-8-yl]-3-(2-hydroxyethyl)urea,
1-[6-(o-chlorophenyl)-1-methyl-4H-s-triazolo[4,3-a][1,4]benzodiazepin-8-yl]-3-(2-hydroxyethyl)-urea,
N-[6-(o-chlorophenyl)-1-methyl-4H-s-triazolo[4,3-a][1,4]benzodiazepin-8-yl]-1-pyrrolidincarboxamide and
N-[6-(o-fluorophenyl)-1,4,4-trimethyl-4H-s-triazolo[4,3-a][1,4]benzodiazepin-8-yl]-1-pyrrolidincarboxamide.

13. Compounds of the general formula

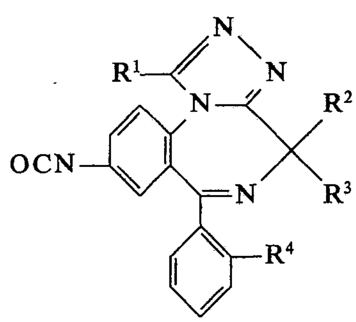

(II)

wherein $R^1$, $R^2$, $R^3$ and $R^4$ have the significance given in claim 1.

14. Compounds of the general formula

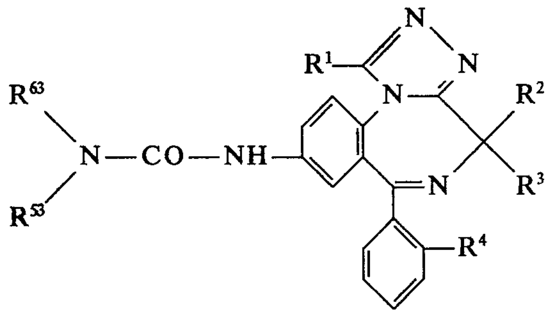

(VII)

wherein $R^1$, $R^2$, $R^3$ and $R^4$ have the significance given in claim 1 and either $R^{53}$ signifies a protecting group and $R^{63}$ signifies $C_{1-7}$-alkyl or a residue of the formula

$$—A—O—Y \qquad (VIII)$$

in which A signifies $C_{2-7}$-alkylene and Y signifies a protecting group, or $R^{53}$ signifies hydrogen or $C_{1-7}$-alkyl and $R^{63}$ signifies a residue of formula VIII above.

15. Compounds of the general formula

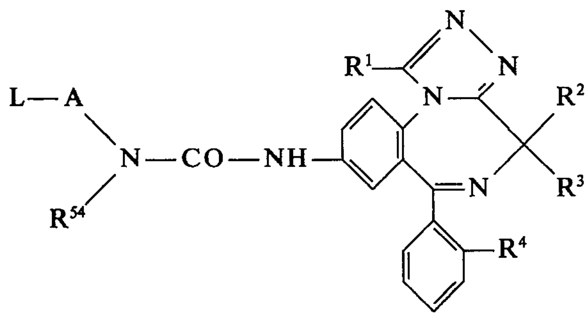

(IX)

wherein $R^1$, $R^2$, $R^3$ and $R^4$ have the significance given in claim 1, and $R^{54}$ signifies hydrogen or $C_{1-7}$-alkyl, L signifies a leaving group and A signifies $C_{2-7}$-alkylene.

16. Compounds according to any one of claims 1 to 12 as pharmaceutically active substances.

17. Compounds according to any one of claims 1 to 12 as aldosterone-antagonistic active substances.

18. Process for the manufacture of compounds according to any one of claims 1 to 12, characterized by

a) reacting a benzodiazepine derivative of the general formula

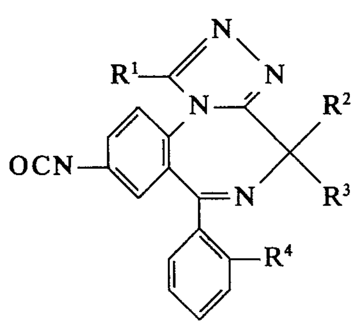

(II)

wherein $R^1$, $R^2$, $R^3$ and $R^4$ have the significance given in claim 1, with an amino compound of the general formula

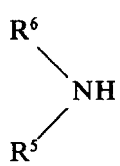

(III)

wherein $R^5$ and $R^6$ have the significance given in claim 1,
or

b) reacting a benzodiazepine derivative of the general formula

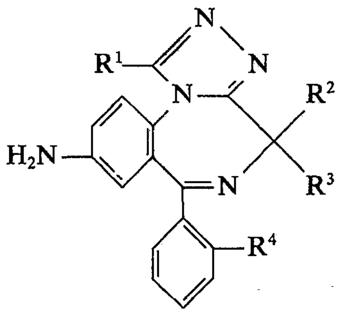

(IV)

wherein $R^1$, $R^2$, $R^3$ and $R^4$ have the significance given in claim 1, with a halide of the general formula

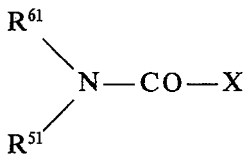

(V)

wherein X signifies halogen and either $R^{51}$ and $R^{61}$ each signifies $C_{1-7}$-alkyl or $R^{51}$ and $R^{61}$ together with the nitrogen atom signify a 3- to 7-membered heterocycle,
or

c) reacting a benzodiazepine derivative of general formula IV above with an isocyanate of the general formula

$R^{62}$—NCO (VI)

wherein $R^{62}$ signifies $C_{1-7}$-alkyl,
or

d) removing the protecting group(s) from a benzodiazepine derivative of the general formula

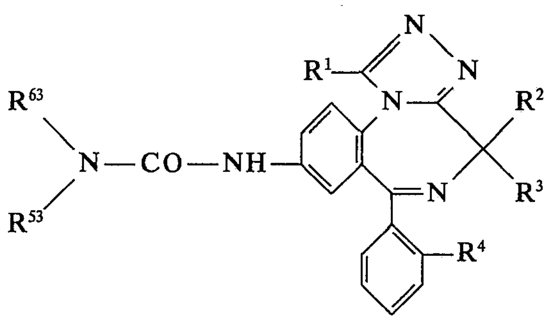

(VII)

wherein $R^1$, $R^2$, $R^3$ and $R^4$ have the significance given in claim 1 and either $R^{53}$ signifies a protecting group and $R^{63}$ signifies $C_{1-7}$-alkyl or a residue of the formula

—A—O—Y (VIII)

in which A signifies $C_{2-7}$-alkylene and Y signifies a protecting group, or $R^{53}$ signifies hydrogen or $C_{1-7}$-alkyl and $R^{63}$ signifies a residue of formula VIII above,
or

e) converting a benzodiazepine derivative of the general formula

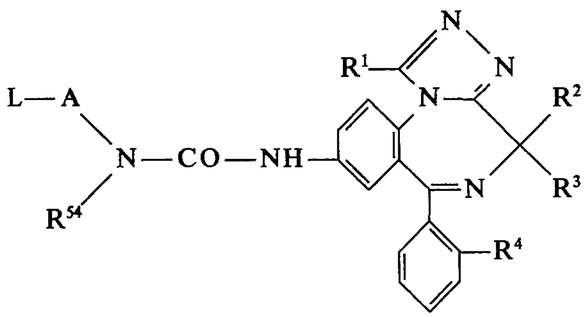

(IX)

wherein $R^1$, $R^2$, $R^3$ and $R^4$ have the significance given in claim 1 and A has the above significance and $R^{54}$ signifies hydrogen or $C_{1-7}$-alkyl and L signifies a leaving group, into the corresponding hydroxy compound, or

f) hydrolytically opening the aziridine ring in a benzodiazepine derivative of the general formula

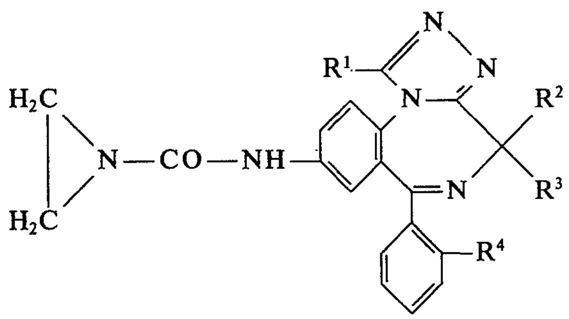

(Ia)

wherein $R^1$, $R^2$, $R^3$ and $R^4$ have the significance given in claim 1, or

g) converting a benzodiazepine derivative of general formula I defined in claim 1 into a pharmaceutically acceptable acid addition salt.

19. Medicament, containing a compound according to any one of claims 1 to 12 or a pharmaceutically acceptable acid addition salt thereof.

20. Aldosterone-antagonistic medicament, containing a compound according to any one of claims 1 to 12 or a pharmaceutically acceptable acid addition salt thereof.

## Revendications

1. Dérivés de benzodiazépine de formule générale

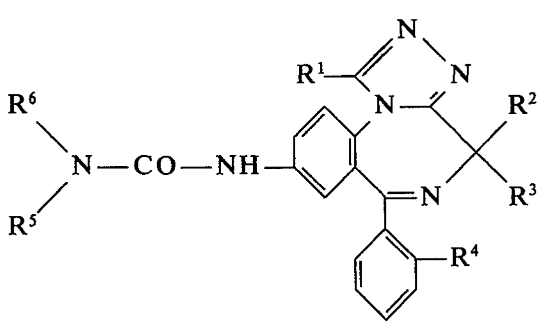

(I)

où $R^1$ représente un hydrogène ou un alcoyle en $C_1$ à $C_7$, $R^2$ et $R^3$ représentent chacun un hydrogène ou un alcoyle en $C_1$ à $C_7$ et $R^4$ représente un halogène et ou bien $R^5$ représente un hydrogène ou un alcoyle en $C_1$ à $C_7$ et $R^6$ représente un alcoyle en $C_1$ à $C_7$ ou un hydroxyalcoyle en $C_2$ à $C_7$, ou $R^5$ et $R^6$ représentent ensemble avec l'atome d'azote un hétérocycle à 3 à 7 chaînons,

et les sels d'addition d'acides pharmaceutiquement acceptables des composés de formule I.

2. Composés selon la revendication 1, caractérisés en ce que $R^1$ représente un hydrogène ou un méthyle.

3. Composés selon l'une des revendications 1 ou 2, caractérisés en ce que $R^2$ et $R^3$ représentent tous deux un hydrogène ou tous deux un méthyle.

4. Composés selon l'une des revendications 1 à 3, caractérisés en ce que $R^4$ représente un fluor ou un chlore.

5. Composés selon l'une des revendications 1 à 4, caractérisés en ce que $R^5$ et $R^6$ représentent tous deux un méthyle ou $R^5$ un hydrogène et $R^6$ un hydroxyéthyle, ou $R^5$ et $R^6$ représentent ensemble avec l'atome d'azote un pyrrolidinyle.

6. 1 - [6 - (o - fluorophényl) - 1,4,4 - triméthyl - 4H - s - triazolo[4,3 - a][1,4]benzodiazépin - 8 - yl]-3-(2-hydroxyéthyl)-urée.

7. 1-[6-(o-chlorophényl)-1,4,4-triméthyl-4H-s-triazolo[4,3-a][1,4]benzodiazépin-8-yl]-3-(2-hydroxyéthyl)-urée.

8. 3-[6-(o-fluorophényl)-4,4-diméthyl-4H-s-triazolo[4,3-a][1,4]benzodiazépin-8-yl]-1,1-diméthylurée.

9. 3 - [6 - (o - chlorophényl) - 4H - s - triazolo[4,3 - a][1,4]benzodiazépin - 8 - yl] - 1,1 - diméthylurée.

10. 3-[6-(o-chlorophényl)-1-méthyl-4H-s-triazolo[4,3-a][1,4]benzodiazépin-8-yl]-1,1-diméthylurée.

11. N-[6-(o-chlorophényl)-4,4-diméthyl-4H-s-triazolo[4,3-a][1,4]benzodiazépin-8-yl]-1-pyrrolidinylcarboxamide.

12. 1-[6-(o-fluorophényl)-4H-s-triazolo[4,3-a][1,4]benzodiazépin-8-yl]-3-(2-hydroxyéthyl)-urée,
3-[6-(o-fluorophényl)-4H-s-triazolo[4,3-a][1,4]benzodiazépin-8-yl]-1,1-diméthylurée,
1-[6-(o-fluorophényl)-1-méthyl-4H-s-triazolo[4,3-a][1,4]benzodiazépin-8-yl]-3-(2-hydroxyéthyl)-urée,
3-[6-(o-fluorophényl)-1,4,4-triméthyl-4H-s-triazolo[4,3-a][1,4]benzodiazépin-8-yl]-1,1-diméthylurée,
1 - [6 - (o - chlorophényl) - 4H - s - triazolo[4,3 - a][1,4]benzodiazépin - 8 - yl] - 3 - (2 - hydroxyéthyl)-urée,
1-[6-(o-chlorophényl)-1-méthyl-4H-s-triazolo[4,3-a][1,4]benzodiazépin-8-yl]-3-(2-hydroxyéthyl)-urée,
N-[6-(o-chlorophényl)-1-méthyl-4H-s-triazolo[4,3-a][1,4]benzodiazépin-8-yl]-1-pyrrolidinecarboxamide et
N-[6-(o-fluorophényl)-1,4,4-triméthyl-4H-s-triazolo[4,3-a][1,4]benzodiazépin-8-yl]-1-pyrrolidinecarboxamide.

13. Composés de formule générale

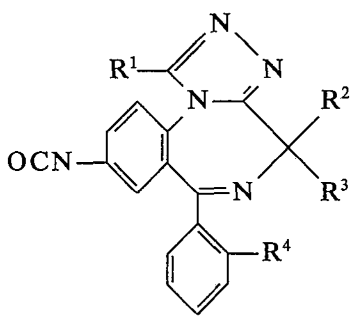

(II)

où $R^1$, $R^2$, $R^3$ et $R^4$ ont la signification donnée dans la revendication 1.

14. Composés de formule générale

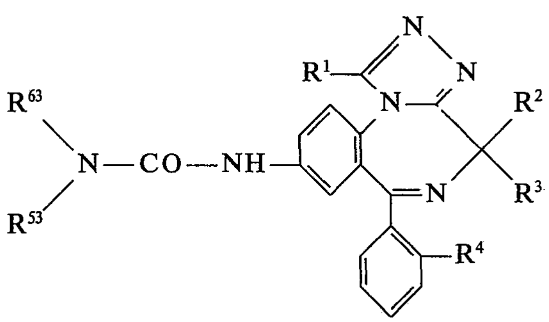

(VII)

où $R^1$, $R^2$, $R^3$ et $R^4$ ont la signification donnée dans la revendication 1 et où ou bien $R^{53}$ représente un groupe protecteur et $R^{63}$ un alcoyle en $C_1$ à $C_7$ ou un radical de formule

$$—A—O—Y \qquad (VIII)$$

où A représente un alcoylène en $C_2$ à $C_7$ et Y un groupe protecteur, ou bien $R^{53}$ représente un Hydrogène ou un alcoyle en $C_1$ à $C_7$ et $R^{63}$ un radical de formule VIII ci-dessus.

15. Composés de formule générale

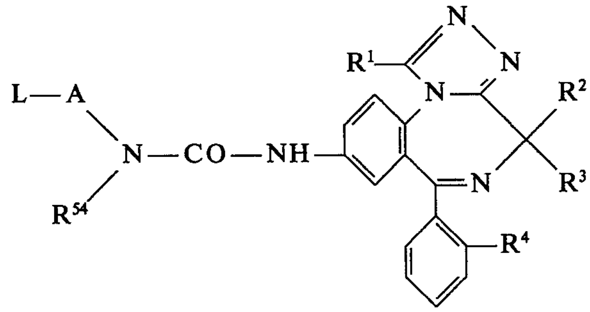

(IX)

où $R^1$, $R^2$, $R^3$ et $R^4$ ont la signification donnée dans la revendication 1, et $R^{54}$ représente un hydrogène ou un alcoyle en $C_1$ à $C_7$, L un groupe sortant et A un alcoylène en $C_2$ à $C_7$.

16. Composés selon l'une des revendications 1 à 12 comme substances actives pharmaceutiques.

17. Composés selon l'une des revendications 1 à 12 comme substances actives antagonistes de l'aldostérone.

18. Procédé de préparation de composés selon l'une des revendications 1 à 12, caractérisé en ce que

a) on fait réagir un dérivé de benzodiazépine de formule générale

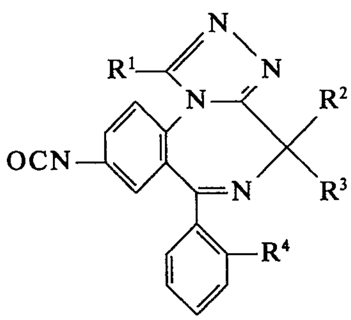

(II)

où $R^1$, $R^2$, $R^3$ et $R^4$ ont la signification donnée dans la revendication 1, avec un composé amino de formule générale

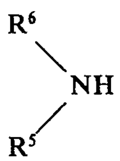

(III)

où $R^5$ et $R^6$ ont la signification donnée dans la revendication 1, ou

b) on fait réagir un dérivé de benzodiazépine de formule générale

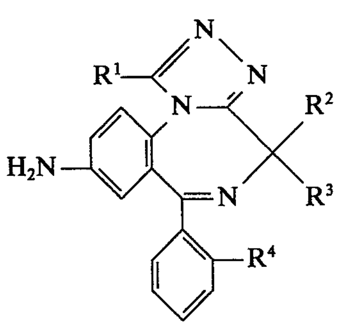

(IV)

où $R^1$, $R^2$, $R^3$ et $R^4$ ont la signification donnée dans la revendication 1, avec un halogénure de formule générale

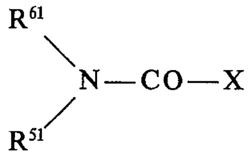

(V)

où X représente un halogène et où ou bien $R^{51}$ et $R^{61}$ représentent chacun un alcoyle en $C_1$ à $C_7$ ou $R^{51}$ et $R^{61}$ forment ensemble avec l'atome d'azote un hétérocycle à 3 à 7 chaînons, ou

c) on fait réagir un dérivé de benzodiazépine de formule générale IV ci-dessus avec un isocyanate de formule générale

$R^{62}$—NCO (VI)

où $R^{62}$ représente un alcoyle en $C_1$ à $C_7$, ou

d) on retire le(s) groupe(s) protecteur(s) d'un dérivé de benzodiazépine de formule générale

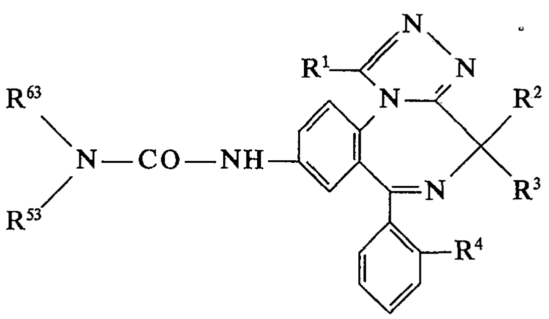

(VII)

où $R^1$, $R^2$, $R^3$ et $R^4$ ont la signification donnée dans la revendication 1 et où ou bien $R^{53}$ représente un groupe protecteur et $R^{63}$ un alcoyle en $C_1$ à $C_7$ ou un radical de formule

—A—O—Y (VIII)

où A représente un alcoylène en $C_2$ à $C_7$ et Y un groupe protecteur, ou bien où $R^{53}$ représente un hydrogène ou un alcoyle en $C_1$ à $C_7$ et $R^{63}$ un radical de formule VIII ci-dessus, ou

e) on transforme un dérivé de benzodiazépine de formule générale

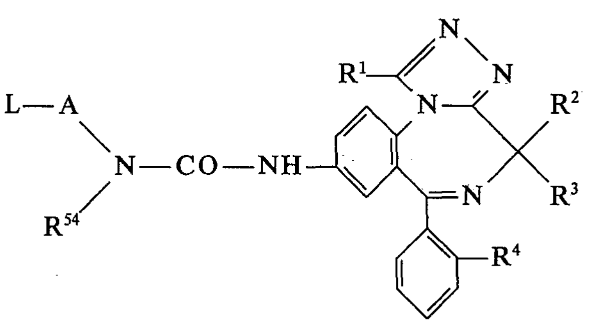

(IX)

où $R^1$, $R^2$, $R^3$ et $R^4$ ont la signification donnée dans la revendication 1 et A a la signification donnée ci-dessus et $R^{54}$ représente un hydrogène ou un alcoyle en $C_1$ à $C_7$ et L un groupe sortant, pour donner le composé hydroxy correspondant, ou

f) dans un dérivé de benzodiazépine de formule générale

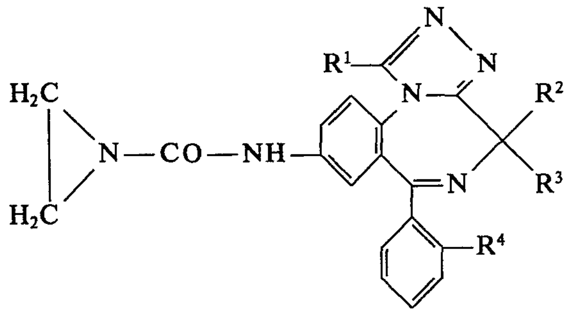

(Ia)

où $R^1$, $R^2$, $R^3$ et $R^4$ ont la signification donnée dans la revendication 1, on ouvre le noyau aziridine par hydrolyse, ou

g) on transforme un dérivé de benzodiazépine de formule générale I définie dans la revendication 1 en un sel d'addition d'acide pharmaceutiquement acceptable.

19. Médicament contenant un composé selon l'une des revendications 1 à 12 ou un de ses sels d'addition d'acides pharmaceutiquement acceptables.

20. Médicament antagoniste de l'aldostérone, contenant un composé selon l'une des revendications 1 à 12 ou un de ses sels d'addition d'acides pharmaceutiquement acceptables.